# EUROPEAN PATENT SPECIFICATION

(11) **EP 3 058 375 B1**
(45) Date of publication and mention of the grant of the patent: **26.12.2018**
(21) Application number: 14792413.8
(22) Date of filing: 15.10.2014
(51) Int. Cl.: G01N 33/84, A61B 5/055, A61K 49/06, G01N 24/08, G01R 33/56, A61K 49/10, G01R 33/28, G01R 33/465, A61B 5/145

(54) **PH-BIOSENSORS BASED ON COMPOUNDS PRODUCED FROM PYRUVIC ACID FOR MAGNETIC RESONANCE IMAGING AND SPECTROSCOPY AND THEIR USES**
PH-BIOSENSOREN AUF DER BASIS VON VERBINDUNGEN AUS BRENZTRAUBENSÄURE FÜR MAGNETRESONANZBILDGEBUNG UND SPEKTROSKOPIE SOWIE DEREN VERWENDUNGEN
BIOCAPTEURS DE PH À BASE DE COMPOSÉS PRODUITS À PARTIR D'ACIDE PYRUVIQUE POUR IMAGERIE ET SPECTROMÉTRIE PAR RÉSONANCE MAGNÉTIQUE ET LEURS UTILISATIONS

(30) Priority: 15.10.2013 EP 13188679; 04.04.2014 EP 14163609
(43) Date of publication of application: 24.08.2016
(73) Proprietor: Technische Universität München, 80333 München (DE)
(72) Inventor: SCHILLING, Franz, 82418 Seehausen am Staffelsee (DE); GLASER, Steffen, 85748 Garching (DE); DÜWEL, Stephan, 80779 Munich (DE); GERSCH, Malte, 67346 Speyer (DE)
(74) Representative: Engelhard, Markus
(86) International application number: PCT/EP2014/072137
(87) International publication number: WO 2015/055727

(56) References cited:
- WO-A2-2006/011810
- WO-A2-2008/020765
- US-B1- 6 596 258
- ALBERT P. CHEN ET AL: "Simultaneous investigation of cardiac pyruvate dehydrogenase flux, Krebs cycle metabolism and pH, using hyperpolarized [1,2-13C2]pyruvate in vivo", NMR IN BIOMEDICINE, vol. 25, no. 2, 19 February 2012 (2012-02-19), pages 305-311, XP055087212, ISSN: 0952-3480, DOI: 10.1002/nbm.1749
- MERRITT M E ET AL: "Hyperpolarized 13C allows a direct measure of flux through a single enzyme-catalyzed step by NMR", PROCEEDINGS OF THE NATIONAL ACADEMY OF SCIENCES, NATIONAL ACADEMY OF SCIENCES, US, vol. 104, no. 50, 11 December 2007 (2007-12-11), pages 19773-19777, XP002573259, ISSN: 0027-8424, DOI: 10.1073/PNAS.0706235104 [retrieved on 2007-12-03]
- X. ZHANG ET AL: "Tumor pH and Its Measurement", THE JOURNAL OF NUCLEAR MEDICINE, vol. 51, no. 8, 21 July 2010 (2010-07-21), pages 1167-1170, XP055104073, ISSN: 0161-5505, DOI: 10.2967/jnumed.109.068981

## Description

The present invention relates to the use of compounds with at least one pH-sensitive ¹³C chemical shift for determining pH and/or measuring pH changes in magnetic resonance. More specifically, the present disclosure is related to compounds with at least one pH-sensitive ¹³C chemical shift, such compound being selected from compounds produced from pyruvic acid after interaction with acid and wherein the compound exhibits at least one pH-sensitive ¹³C chemical shift in an NMR spectrum. The present disclosure further relates to biosensors comprising at least one of the compounds. The present invention is furthermore related to *in vitro* and *in vivo* methods for determining pH and/or measuring pH changes using the compounds.

### BACKGROUND OF THE INVENTION

In mammalian tissues, intra- and extracellular pH are regulated in a dynamic steady state driven by metabolic acid production, export of H⁺ from cells, and diffusion of these H⁺ equivalents from the site of production to the blood, where they are buffered by an open and dynamic CO₂/HCO₃⁻ system. Although this balance is quite robust, it can be altered in many pathological states, notably cancers, renal failure, ischemia, inflammation and chronic obstructive pulmonary disease (Gillies et al., 2004).

In the field of magnetic resonance various pH -sensor molecules have been developed whose ¹H, ¹⁹F, or ³¹P resonance frequencies (chemical shifts) change with pH (Gillies et al., 2004; Arnold et al., 1984; De Leon et al., 2009; Morikawa et al., 1993 and Zhang et al., 2010). Those methods allow for a non-invasive detection of both intra- and extracellular pH. However, they suffer from low sensitivity and are thus not suitable for highly spatially resolved pH mapping by magnetic resonance imaging (MRI).

For this reason other classes of exogenous pH-sensitive contrast agents were developed based on pH-dependent magnetization transfer between water and a contrast agent (mostly lanthanoid complexes) or based on pH-dependent relaxation properties of gadolinium complexes (Gillies et al., 2004; De Leon et al., 2009; Aime et al., 2002; Castelli et al, 2013). The main disadvantages of these pre-clinically applied methods are that they require either long irradiation with radiofrequency waves or an exact determination of contrast agent concentration. Therefore, it is unclear, whether those techniques will translate into clinical applications. Long radiofrequency irradiation is mostly prohibited by specific absorption rate (SAR) limitations in the clinic and gadolinium-/lanthanoid-complexes are restricted in clinical use due to their toxicity.

In 2003 dissolution dynamic nuclear polarization (DNP) revolutionized magnetic resonance spectroscopy by bringing nuclear spins in a so-called hyperpolarized state leading to a sensitivity gain by more than four orders of magnitude. This allows to image formerly insensitive nuclei such as ¹³C (Ardenkjaer-Larsen et al., 2003). A technique for mapping pH spatially by taking the ratio of hyperpolarized bicarbonate (HCO₃⁻) to CO₂ also relies on DNP which represents the current state-of-the-art method in NMR-based pH measurements (Gallagher et al., 2008). Disadvantages of this method are the signal-to-noise-ratio-limited accuracy in the measurement of peak intensities and the influence of enzyme concentration (e.g. carbonic anhydrase) on the measurement of pH (Schroeder et al., 2010).

Hyperpolarized [1-¹³C]-pyruvate is described to be used for detecting tumor response to chemotherapy treatment in lymphoma-bearing mice (Day et al., 2007) and is currently being used in patients as a novel contrast agent in a clinical study at the University of San Francisco for applications in metabolic imaging of prostate carcinoma (Nelson et al., 2013 -1 and Nelson et al., 2013 -2). This first-in-man imaging study evaluated the safety and feasibility of hyperpolarized [1-¹³C]-pyruvate as an agent for noninvasively characterizing alterations in tumor metabolism for patients with prostate cancer. It was possible to evaluate the distribution of [1-¹³C]-pyruvate and its metabolic product lactate in a matter of seconds, as well as the flux of pyruvate to lactate.

WO 2008/020764 A1 discloses methods of ¹³C-MR imaging and/or ¹³C-MR spectroscopy of cell death using an imaging medium which comprises hyperpolarized ¹³C-pyruvate. WO 2008/020765 A2 discloses an imaging medium containing lactate and hyperpolarized ¹³C-pyruvate, a method to produce said imaging medium, use of said imaging medium and methods of ¹³C-MR imaging and/or ¹³C-MR spectroscopy wherein said imaging medium is used. WO 2011/138269 A1 discloses a hyperpolarized MR imaging medium comprising hyperpolarized [¹³C, ²H]lactate and a method of ¹³C-MR detection for the determination of lactate dehydrogenase (LDH) activity.

WO 2006/011810 A2 discloses the use of hyperpolarized ¹³C-pyruvate as MR imaging agent. Zhang et al. (2010) disclose the use of hyperpolarized ¹³C-bicarbonate as pH indicator to detect lymphoma xenografts. Thereby, the absolute signal intensities of bicarbonate and CO₂ as quotient are set in relation to the pH value. US 6,596,258 B1 discloses the use of imidazole compounds in a method of obtaining extracellular or intracellular pH images in biological systems by magnetic resonance.

Besides magnetic resonance, optical methods such as fluorescence microscopy (Hassan et al., 2007) or radioactive tracers (Vavere et al., 2009) in positron-emission-tomography (PET) can potentially be used for pH-mapping.

Although many non-invasive pH-mapping methods exist, none of these made the translation from preclinical studies to the clinic.

There is a need in the art for improved means and methods for measuring pH and/or pH changes, preferably in real-time and/or in a spatial resolution, especially *in vivo.*

### SUMMARY OF THE INVENTION

According to the present invention the object is solved as claimed in the claims.

According to the present invention this object is solved by the use of a compound with at least one pH-sensitive ¹³C chemical shift for determining pH and/or measuring pH changes by ¹³C magnetic resonance,
wherein the compound is selected from
zymonic acid,
(*Z*)-4-methyl-2-oxopent-3-enedioic acid (OMPD),
analogs of zymonic acid,
analogs of OMPD,
and their hydrates, salts, solutions, stereoisomers,
wherein an analog of zymonic acid or OMPD is selected from an ester, an ether, an amide, a fluorinated analog, deuterated analog or wherein the zymonic acid analog is selected from 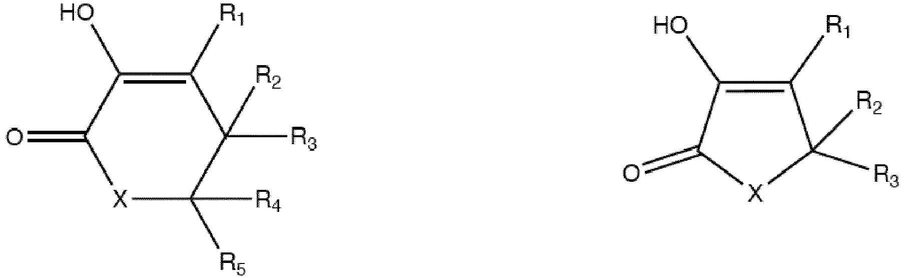
wherein X is selected from CR₆R₇, O, NR₆, S, and wherein R₁ to R₇ is, at each occurrence, independently selected from H, alkyl, halogen, CN, methoxy, carboxy, aryl, e.g. benzyl, wherein, preferably, one of R₂ and R₃ is carboxy.

According to the present invention this object is solved by a method of *in vivo* imaging by magnetic resonance imaging (MRI) or magnetic resonance spectroscopy (MRS) using the compound of the present invention.

According to the present invention this object is solved by providing the compound of the present invention for use in an *in vivo* method for diagnosing and/or monitoring treatment of a disease causing changes in pH by ¹³C magnetic resonance.

According to the present invention this object is solved by using the compound of the present invention as pH sensor for *in vitro* diagnosing and/or monitoring treatment of a disease causing changes in pH by ¹³C magnetic resonance.

According to the present invention this object is solved by using the compound of the present invention as pH sensor for use in *in vitro* magnetic resonance imaging (MRI) or magnetic resonance spectroscopy (MRS).

According to the present invention this object is solved by an *in vitro* method for determining pH and/or measuring pH changes, preferably in real-time, comprising the steps of
(i) providing a sample,
(ii) adding a compound with at least one pH-sensitive chemical shift of the present invention,
(iii) performing magnetic resonance imaging (MRI) or magnetic resonance spectroscopy (MRS) and thereby determining the pH or pH changes of or in the sample by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift, preferably over time.

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

### pH biosensors based on compounds produced from pyruvic acid after interaction with acid

As discussed above, the present invention provides the use of a compound with at least one pH-sensitive ¹³C chemical shift for determining pH and/or measuring pH changes by ¹³C magnetic resonance.

Said compound is selected from
zymonic acid,
(*Z*)-4-methyl-2-oxopent-3-enedioic acid (OMPD),
analogs of zymonic acid,
analogs of OMPD,
and their hydrates, salts, solutions, stereoisomers,
wherein an analog of zymonic acid or OMPD is selected from an ester, an ether, an amide, a fluorinated analog, deuterated analog or wherein the zymonic acid analog is selected from 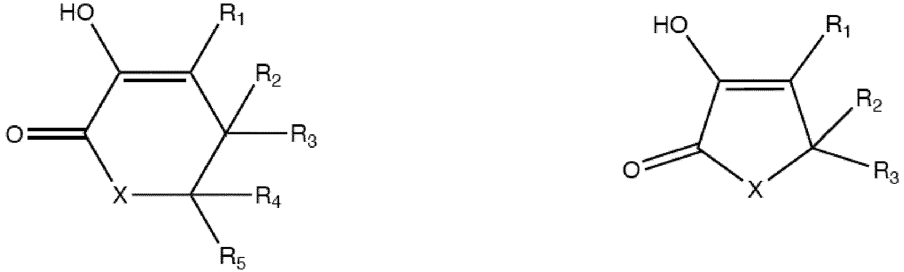
wherein X is selected from CR₆R₇, O, NR₆, S, and wherein R₁ to R₇ is, at each occurrence, independently selected from H, alkyl, halogen, CN, methoxy, carboxy, aryl, e.g. benzyl, wherein, preferably, one of R₂ and R₃ is carboxy.

The inventors have surprisingly found that it is possible to make use of the pH-dependent displacement of ¹³C chemical shifts in compounds for determining one or several pH values and/or for measuring pH changes. In particular, this concerns the displacement of pH sensitive ¹³C chemical shifts in ¹³C-magnetic resonance imaging and/or ¹³C magnetic resonance spectroscopy.

The present invention thus provides for the use of a compound which shows a pH-dependent displacement of at least one pH-sensitive ¹³C chemical shift for determining pH and/or measuring pH changes. The term "displacement of a chemical shift, ", as used herein is meant to refer to a change in position of the respective chemical shift. In this context, "displacement of a chemical shift" is preferably meant to refer to a change in position of a ¹³C chemical shift.

Preferably, a compound with at least one pH-sensitive chemical shift comprises one or more pH-sensitive chemical shifts, such as two, three, four or more.

Here, a novel pH biosensor is presented (that is based on a compound with at least one pH-sensitive ¹³C chemical shift, namely zymonic acid, its analogs, or OMPD ((*Z*)-4-methyl-2-oxopent-3-enedioic acid), its analogs, as defined herein) for magnetic resonance that is very sensitive to pH-changes in a physiologically and/or pathologically relevant pH range. This novel sensor acts independently of its concentration and enzymatic reactions and therefore allows a very accurate pH mapping at high spatial resolution making it a promising probe for the translation to the clinic.

As used herein "magnetic resonance" refers to the observation of Larmor precession in a magnetic field (see Ernst, 1997 and de Graaf, 2007), and includes measurements at a NMR spectrometer, an NMR microimaging system, an MRI scanner, a low-field NMR device, microfluidic arrays ("NMR on a chip"), and/or combinations thereof. Measurement includes all variations of spatially and/or spectrally resolved magnetic resonance techniques, such as magnetic resonance spectroscopy (MRS), magnetic resonance imaging (MRI), or magnetic resonance spectroscopic imaging (MRSI).

As used herein "chemical shift" in magnetic resonance refers to the resonance of a nucleus relative to a suitable standard, such as tetramethylsilane (TMS).

The compounds comprise at least one enolic group (preferably one or more enolic groups, such as two, three, four or more) whose pKa-value is lowered through effects of one or more neighboring groups into a physiological and/or pathological pH-range, such as two, three, four or more neighboring groups.

A "neighboring group to a specific group" refers to a functional group (such as a carboxylic group or ester group in zymonic acid) connected up to seven bonds away from such specific group, e.g. from the enolic group.

A "physiological and/or pathological pH-range" or "physiologically and/or pathologically relevant pH-range" refers to pH ranges of from about 5 to about 9. Preferably, a "physiological pH-range" is from about 6 to about 8.

According to the present invention, said compound with at least one pH-sensitive ¹³C chemical shift exhibits at least one NMR resonance with a pH-sensitive chemical shift in an NMR spectrum.

Preferably, the compounds of the present invention are produced from pyruvic acid or pyruvate after interaction with acid.

### - Zymonic acid

According to the invention, one compound with at least one pH-sensitive ¹³C chemical shift is zymonic acid.

Zymonic acid is also referred to as 2,5-dihydro-4-hydroxy-2-methyl-5-oxo-2-furancarboxylic acid.
IUPAC name: 4-hydroxy-2-methyl-5-oxo-2,5-dihydrofuran-2-carboxylic acid
Traditional IUPAC name: 4-hydroxy-2-methyl-5-oxofuran-2-carboxylic acid
(see http://www.hmdb.ca/metabolites/HMDB31210))

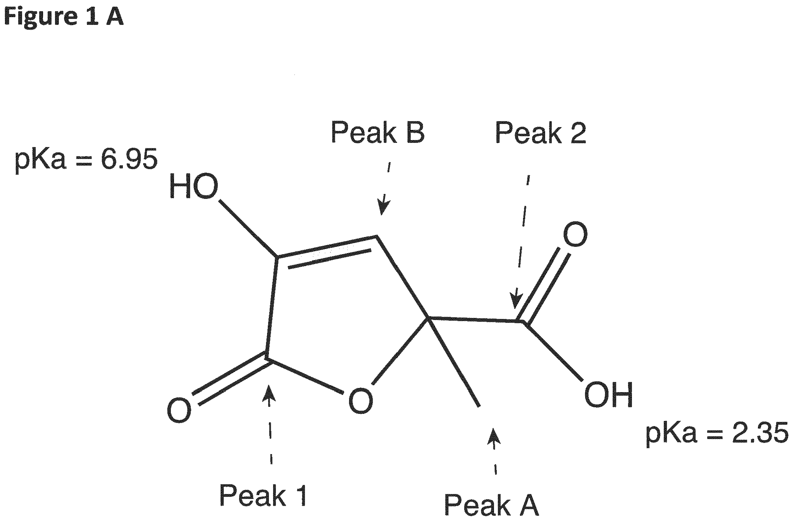

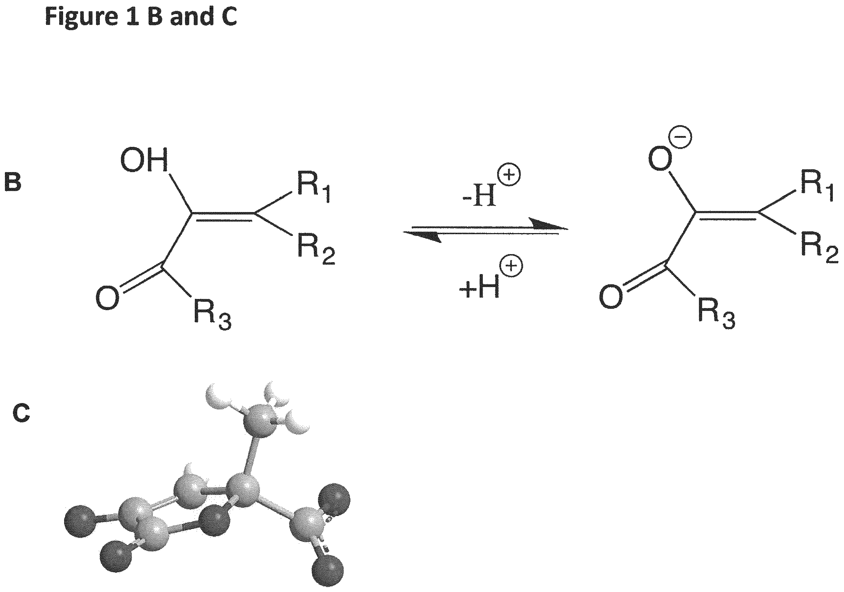

The inventors have found that zymonic acid exhibits a pH-dependent chemical shift for some of its ¹³C- and ¹H-resonances (marked in Figure 1). In Figure 1, zymonic acid's pKₐ-values are shown and assigned to the relevant proton donating groups. The pKₐ of the enolic group is 6.95.

Zymonic acid has been mentioned in the 1950s for the first time and can be produced by yeast bacteria from glucose or can originate from a ring closure of parapyruvate molecules which in turn can originate from pyruvic acid (Bloomer et al., 1970 -1; Bloomer et al., 1970 -2; Stodola et al., 1952; de Jong, 1901). Zymonic acid is used as a flavor constituent for confectionary and in the tobacco industry and is therefore not toxic when administered *in vivo.*

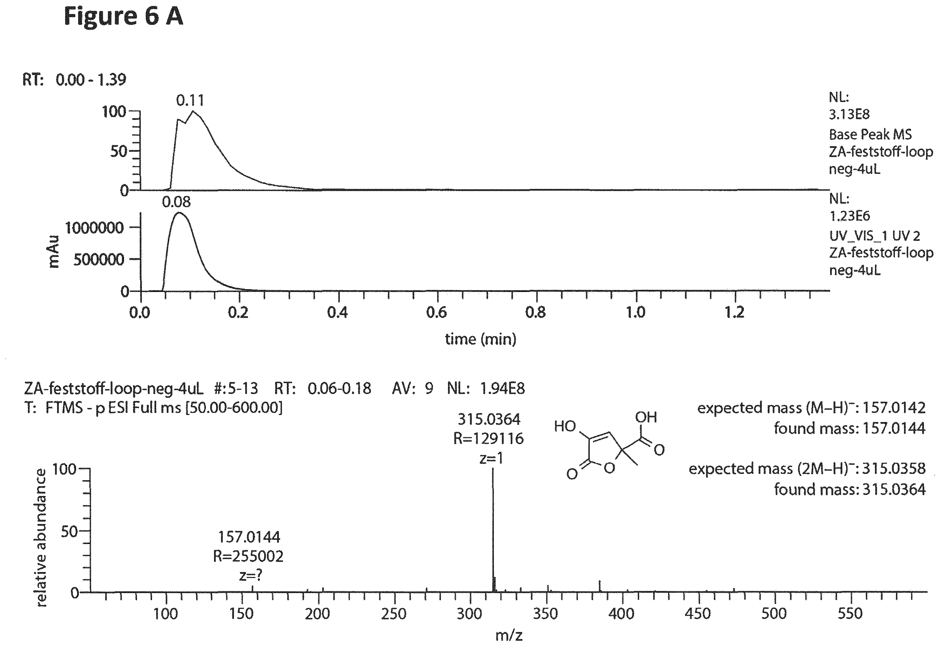

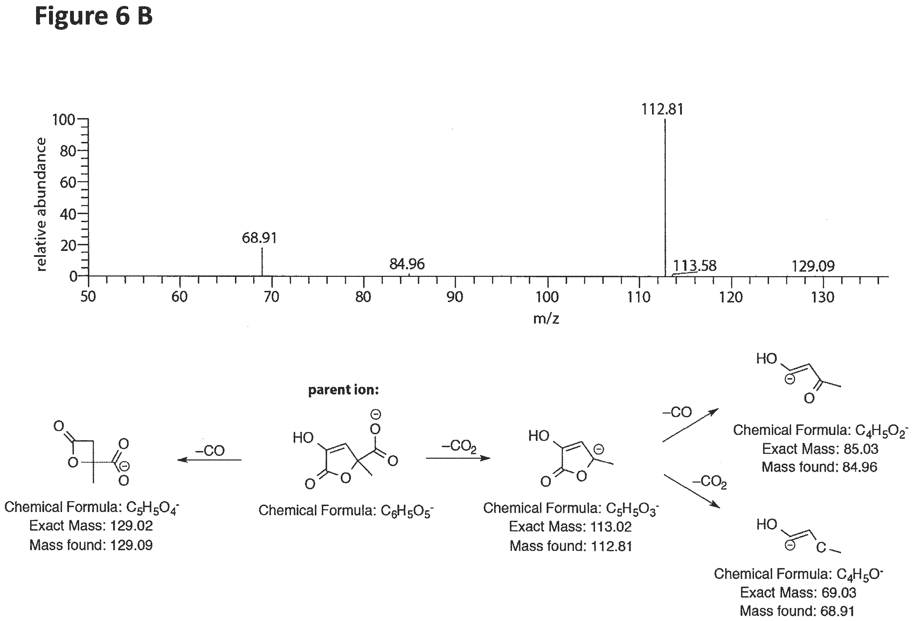

Zymonic acid is an extremely sensitive pH biosensor for magnetic resonance spectroscopy (MRS) and magnetic resonance imaging (MRI) and exhibits the following properties, which sets zymonic acid apart from other non-invasive methods to measure pH:
(a) Zymonic acid exhibits the second highest pH-dependent change in chemical shift measured to date with ¹³C shifts up to 2.35 ppm/pH in the physiologically and/or pathologically relevant range from pH 5 to pH 9 and is, thus, suitable for a very accurate noninvasive pH determination using magnetic resonance spectroscopy/imaging. It should be noted that such pH-dependent change in chemical shift typically is a change in the position of the respective resonance peak of this chemical shift in an NMR spectrum.
(b) Unlike some other pH measurement methods, the pH determination is robustly performed using a relative or absolute frequency encoding. In contrast, the amplitude encoding used in the bicarbonate method (i.e. a change in intensity) (Gallagher et al., 2008; Schroeder et al., 2010) is prone to spatial or temporal fluctuations in concentration.
(c) ¹³C-labeled zymonic acid (see peaks 1 and 2 in Figure 6) can be produced from pyruvate or pyruvic acid (either ¹³C-labeled or not, such as [1-¹³C]-pyruvate) in a one-step synthesis. The two labeled carbons are exposed to a weak dipolar interaction and thus exhibit a long longitudinal relaxation time T₁. Hyperpolarization increases the polarization of the molecule by four to five orders of magnitude which enables pH imaging in the human body at low contrast agent concentration in the micromolar to millimolar concentration range and at the same time high spatial resolution with centimeter to sub-millimeter voxel size.

### - OMPD

According to the invention, one compound with at least one pH-sensitive ¹³C chemical shift is OMPD ((Z)-4-methyl-2-oxopent-3-enedioic acid).

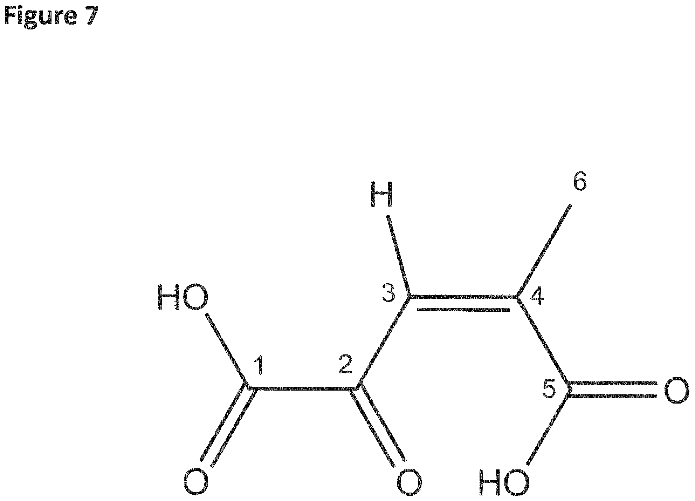

The inventors have found that the chemical compound "(Z)-4-methyl-2-oxopent-3-enedioic acid (OMPD)", which can be produced from pyruvate through catalysis by strong acid, exhibits a pH-dependent chemical shift for its ¹³C- and ¹H-resonances (see Figure 7).

OMPD was first discovered in tulip plants in 1988 (Ohyama et al., 1988-1; Ohyama et al., 1988-2; Ohyama et al., 2006).

OMPD is an extremely sensitive pH biosensor for magnetic resonance spectroscopy and magnetic resonance tomography and exhibits the following properties, which sets OMPD apart from other non-invasive methods to measure pH:
(a) OMPD exhibits the highest pH-dependent change in chemical shift measured to date with up to 12.2 ppm/pH in the physiological and pathologically relevant range from pH 5 to pH 9 and is thus suitable for a very accurate noninvasive pH determination using magnetic resonance tomography.
(b) Unlike other pH measurement methods, the pH determination is robustly performed using a relative or absolute frequency encoding. (To this end, one can either determine the difference between two chemical shifts within OMPD itself as a function of pH or the difference of one or several chemical shifts of OMPD with regard to a pH independent reference/reference compound, e.g. urea, added to the sample.) In contrast, the amplitude encoding used in the bicarbonate method is prone to spatial or temporal fluctuations in concentration, as discussed above.
(c) ¹³C-labeled OMPD can be produced from pyruvate or pyruvic acid (either ¹³C-labeled or not, such as [1-¹³C]-pyruvate) in a one-step synthesis. The two carboxyl groups are exposed to a weak dipolar interaction and thus exhibit a long longitudinal relaxation time T₁. Hyperpolarization increases the polarization of the molecule by four to five orders of magnitude, which enables pH imaging in the human body at low contrast agent concentration in the micromolar to millimolar concentration range and at the same time high spatial resolution with centimeter to sub-millimeter voxel size.
(d) OMPD is stable in aqueous solutions.

According to the present invention, the compound is selected from
zymonic acid,
analogs of zymonic acid,
(*Z*)-4-methyl-2-oxopent-3-enedioic acid (OMPD),
analogs of OMPD,
and their hydrates, salts, solutions, stereoisomers.

An "analog of zymonic acid" or an "analog of OMPD" according to the invention is
- an ester, e.g. methyl ester,
- an ether,
- an amide,
- a fluorinated analog, e.g. a trifluoromethyl analog, or a
- deuterated analog, or
the zymonic acid analog is selected from 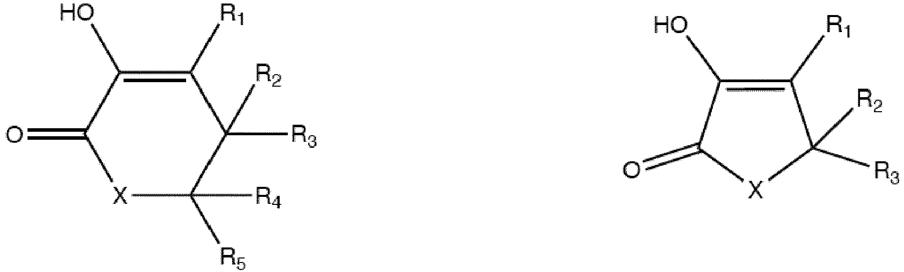 wherein X is selected from CR₆R₇, O, NR₆, S, and wherein R₁ to R₇ is, at each occurrence, independently selected from H, alkyl, halogen, CN, methoxy, carboxy, aryl, e.g. benzyl, wherein, preferably, one of R₂ and R₃ is carboxy.

Enolic acids with a cyclic hydrogen bond are also described, such as 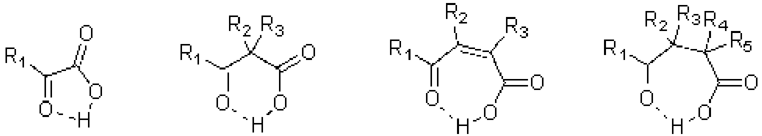 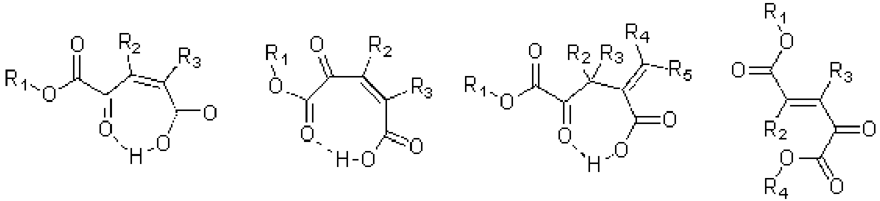 wherein R₁ to R₅, is, at each occurrence, independently selected from H, alkyl (such as methyl), halogen, CN, methoxy, carboxy, aryl, (such as benzyl), amino.

According to the present invention, bicarbonate as a metabolite of pyruvic acid is not encompassed by the invention.

According to the present invention, acetic acid and acetate as metabolites of pyruvic acid are not encompassed by the invention (as described in Jensen et al., 2013).

### - further components

The compound with at least one pH-sensitive chemical shift can comprise further components, such as
- linker, and/or
- modulator fragment(s).

The modulator fragment preferably modulates or controls subcellular localization, cellular uptake, pharmacokinetic properties and/or specific binding to target cells and/or tissue, such as to tumor cells.

The modulator fragment can be coupled via a linker.

### - pH sensitivity

A (acid) compound with at least one pH-sensitive chemical shift used in the invention includes, inter alia, zymonic acid, its analogs, OMPD, its analogs and further compounds, as defined herein.

The compound is ¹³C-labeled.

More preferably, the compound exhibits at least one pH-sensitive ¹³C-chemical shift, such as in the range of 170-180 ppm.

Preferably, the compound exhibits at least one pH-sensitive ¹³C chemical shift sensitive in the physiological and/or pathological pH range, from about pH 5 to about pH 9.

In a preferred embodiment, the compound is hyperpolarized.

Hyperpolarization of NMR active ¹³C-nuclei may be achieved by different methods, which are for instance described in WO 98/30918, WO 99/24080 and WO 99/35508, and hyperpolarization methods are polarization transfer from a noble gas, "brute force", spin refrigeration, the parahydrogen method (parahydrogen induced polarisation (PHIP)) and dynamic nuclear polarization (DNP).

Preferably, the hyperpolarization is by dynamic nuclear polarization (DNP).

The term "hyperpolarized" refers to a nuclear polarization level in excess of 0.1%, more preferred in excess of 1% and most preferred in excess of 10%. The level of polarization may for instance be determined by solid state ¹³C-NMR measurements, such as in solid hyperpolarized ¹³C-zymonic acid or ¹³C-OMPD (or other compounds), e.g. obtained by dynamic nuclear polarization (DNP) of ¹³C-zymonic acid or ¹³C-OMPD. The solid state ¹³C-NMR measurement preferably consists of a simple pulse-acquire NMR sequence using a low flip angle. The signal intensity of the hyperpolarized ¹³C-zymonic acid/OMPD in the NMR spectrum is compared with signal intensity in a NMR spectrum acquired before the polarization process. The level of polarization is then calculated from the ratio of the signal intensities before and after polarization. In a similar way, the level of polarization for dissolved hyperpolarized ¹³C-zymonic acid or ¹³C-OMPD (or other compounds) may be determined by liquid state NMR measurements. Again the signal intensity of the dissolved hyperpolarized ¹³C-zymonic acid or ¹³C-OMPD is compared with the signal intensity before polarization. The level of polarization is then calculated from the ratio of the signal intensities before and after polarization.

Preferably, the (acid) compound with one or more pH-sensitive chemical shifts used in the invention has a pKₐ value in a physiological and/or pathological pH range (from about pH 5 to about pH 9).

Preferably, the carbon(s) belonging to the pH-sensitive chemical shift(s) of the (acid) compound with one or more pH-sensitive chemical shifts exhibit(s) a long longitudinal relaxation time T₁.

### - Chemical shift reference

Preferably, a reference chemical shift which is pH-insensitive, i.e. not pH-sensitive and, thus, exhibits no change in chemical shift upon change of pH, is required. This is typically provided in the form of a further compound, the "reference compound", that is added to or also present in a sample.

Alternatively, a chemical shift with a different chemical-shift-pH-correlation can serve as a reference. This may, e.g., be a chemical shift within the compound according to the present invention.

The reference chemical shift can be an endogenous reference or an exogenous reference or a chemical shift of the compound itself or its metabolites.

In one embodiment, the (acid) compound with one or more pH-sensitive chemical shifts used in the invention furthermore exhibits at least one chemical shift that is not pH-sensitive, preferably at least one pH-insensitive ¹³C-chemical shift, (endogenous reference)

In one embodiment, the (acid) compound with one or more pH-sensitive chemical shifts used in the invention furthermore exhibits at least one chemical shift that is pH-sensitive with a different chemical-shift-pH-correlation, preferably at least one pH-sensitive ¹³C-peak. (endogenous pH-sensitive reference)

In one embodiment, a reference compound is used. The reference compound is a compound which does not exhibit pH-sensitive shift(s) (exogenous reference).

Preferably the reference compound is ¹³C-labeled and preferably exhibits at least one pH-insensitive ¹³C-peak.

A preferred reference compound is ¹³C urea (or ¹³C-pyruvate, or ¹³C-pyruvate hydrate, or ¹³C-parapyruvate, or ¹³C-lactate, or ¹³C-alanine).

For example, the reference compound is obtained in that a substance or compound (such as urea) is co-polarized at the same time when the compound with one or more pH-sensitive chemical shift of the invention is hyperpolarized.

The present disclosure also describes an *imaging medium,* comprising
at least one compound with at least one pH-sensitive chemical shift as defined herein,
optionally, pharmaceutically acceptable carriers and/or excipients, such as an aqueous carrier, like a buffer.

The imaging medium can be a magnetic resonance (MR) imaging medium.

The term "imaging medium" refers to a liquid composition comprising at least one compound with one or more pH-sensitive chemical shifts used in the present invention (such as hyperpolarized ¹³C-zymonic acid or hyperpolarized ¹³C-pyruvate or hyperpolarized ¹³C-OMPD) as the MR active agent. The imaging medium may be used as imaging medium in MR imaging or as MR spectroscopy agent in MR spectroscopy. The imaging medium may be used as imaging medium for *in vivo* MR imaging and/or spectroscopy, i.e. MR imaging and/or spectroscopy carried out on living human or non-human animal beings. Further, the imaging medium may be used as imaging medium for *in vitro* MR imaging and/or spectroscopy, e.g. for determining pH and/or pH changes in cell cultures or *ex vivo* tissues. Cell cultures may be derived from cells obtained from samples derived from the human or non-human animal body, like for instance blood, urine or saliva, while *ex vivo* tissue may be obtained from biopsies or surgical procedures.

For example, the imaging medium comprises in addition to the MR active agent an aqueous carrier, such as a physiologically tolerable and pharmaceutically accepted aqueous carrier, like water, a buffer solution or saline. Such an imaging medium may further comprise conventional pharmaceutical or veterinary carriers or excipients, e.g. formulation aids such as are conventional for diagnostic compositions in human or veterinary medicine.

For example, the imaging medium comprises in addition to the MR active agent a solvent which is compatible with and used for *in vitro* cell or tissue assays, for instance DMSO or methanol or solvent mixtures comprising an aqueous carrier and a non-aqueous solvent, for instance mixtures of DMSO and water or a buffer solution or methanol and water or a buffer solution.

For example, at least one compound with at least one or more pH-sensitive chemical shifts is used in concentrations of up to 1 M, preferably 0.1 to 100 mM, such as 10 to 50 mM, in the imaging medium.

The present disclosure also describes a *biosensor* for determining pH and/or measuring pH changes, comprising
at least one compound with at least one pH-sensitive ¹³C chemical shift as defined herein,
optionally, a reference compound,
optionally, pharmaceutically acceptable carriers and/or excipients.

The biosensor can comprise
(i) a pH sensitive fragment
   comprising or consisting of the at least one compound with at least one pH-sensitive chemical shift as defined herein,
   coupled to (ii), optionally via a linker,
(ii) a modulator fragment.

The modulator fragment (ii) can modulate or controls subcellular localization, cellular uptake, pharmacokinetic properties and/or specific binding to target cells and/or tissue, such as to tumor cells.

For example, the reference compound is a compound which does not exhibit pH-sensitive chemical shift(s) (exogenous reference chemical shift, as described above).

The reference compound can be ¹³C-labeled and can exhibit at least one pH-insensitive ¹³C-chemical shift.

Alternatively, a chemical shift with a different chemical-shift-pH-correlation can serve as a reference (see definition of chemical shift reference above).

An exemplary reference compound is ¹³C urea.

For example, the reference compound is obtained in that a substance or compound (such as urea) is co-polarized at the same time when the compound with one or more pH-sensitive chemical shifts used in the invention is hyperpolarized.

The at least one compound with at least one/one or more pH-sensitive chemical shift can be used in concentrations of up to 1 M, such as 0.1 to 100 mM, such as 10 to 50 mM, in the biosensor.

### Imaging and medical uses

As discussed above, the present invention provides the compound with at least one pH-sensitive chemical shift used in the present invention (the imaging medium of the present invention) for use in *in vivo* magnetic resonance imaging (MRI) or magnetic resonance spectroscopy (MRS).

As discussed above, the present invention provides the compound with at least one pH-sensitive chemical shift used in the present invention (the imaging medium of the present invention) for use in diagnosing and/or monitoring treatment of a disease causing changes in pH. In particular for use
- in an *in vivo* method for diagnosing and/or monitoring treatment of a disease causing changes in pH; or
- as pH sensor for *in vitro* diagnosing and/or monitoring treatment of a disease causing changes in pH.

Thereby, the progress of a disease and/or the treatment of a disease can be monitored. Preferably, a "disease causing changes in pH" is selected from cancers, inflammation, ischemia, renal failure and chronic obstructive pulmonary disease.

Preferably, the imaging is real-time.

Preferably, the uses comprise the resolution of the spatial pH distribution,
preferably, comprising the use of frequency encoding techniques, such as all methods of chemical shift imaging (CSI) and phase sensitive encodings of chemical shifts (as e.g. used in non-invasive spatially resolved temperature measurements using changes in proton resonance frequencies (0.01 ppm/°C)). See Rieke et al., 2004.

As discussed above, the present invention provides the use of the compound of the present invention as pH sensor for *in vitro* NMR-spectroscopy.

Preferably, the use comprises response-to-treatment monitoring of treatments applied to cell lines.

### Methods for determining pH and/or measuring pH changes

As discussed above, the present invention provides an *in vitro* method for determining pH and/or measuring pH changes.

Said *in vitro* method of the present invention comprises the steps of
(i) providing a sample,
(ii) adding a compound with at least one pH-sensitive chemical shift of the present invention to the sample,
(iii) performing magnetic resonance imaging (MRI) or magnetic resonance spectroscopy (MRS) and thereby determining the pH or pH changes of or in the sample by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift,
wherein the sample is a cell culture sample, such as derived from a human or non-human body, *ex vivo* tissue, cell culture, and/or step (iii) is carried out in an MRI scanner machine with MRS or MRSI capabilities or in a NMR spectrometer (such as with a microimaging head).

Preferably, the pH-independent chemical shift (acting as a reference chemical shift) is from the same compound, i.e. the compound with at least one pH-sensitive chemical shift (endogenous reference chemical shift, as described above), or from another substance (exogenous reference chemical shift, as described above), and is used as a pH-independent reference.

Alternatively, a chemical shift with a different chemical-shift-pH-correlation can serve as a reference.

Further described is an *in vivo* method which comprises the steps of
(i) applying or administering a compound with at least one pH-sensitive chemical shift of the present invention, an imaging medium described in the present invention or a biosensor described in the present invention to the body of a patient or non-human animal,
(ii) performing magnetic resonance imaging (MRI) and thereby determining one or several pH values or pH changes of or in the body of said patient or non-human animal by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift.

The *in-vivo* method can be a real-time method.

The patient can be a human.

The patient can be diagnosed with a disease causing changes in pH or the treatment of a disease causing changes in pH can be monitored.

"A disease causing changes in pH" can be selected from cancers, inflammation, ischemia, renal failure and chronic obstructive pulmonary disease.

The pH-independent chemical shift (reference chemical shift) can be from the same compound, i.e. the compound with at least one pH-sensitive chemical shift (endogenous reference chemical shift, as described above), or from another substance (exogenous reference chemical shift, as described above), and is used as a pH-independent reference.

Alternatively, a chemical shift with a different chemical-shift-pH-correlation can serve as a reference.

Preferably, the *in vitro* method according to the invention comprises the resolution of the spatial pH distribution and, thus, obtaining spatially resolved NMR spectra,
preferably, comprising the use of frequency encoding techniques, such as all methods of chemical shift imaging (CSI) and phase sensitive encodings of chemical shifts (as e.g. used in non-invasive spatially resolved temperature measurements using changes in proton resonance frequencies (0.01 ppm/°C)). See Rieke et al., 2004.

### Methods for diagnosing and/or monitoring treatment

Further described is a method of diagnosing and/or monitoring treatment of a disease causing changes in pH.

Said method comprises the steps of
(i) applying or administering a compound with at least one pH-sensitive chemical shift of the present invention (an imaging medium as described herein) or a biosensor described in the present disclosure to the body of a patient or non-human animal,
(ii) performing magnetic resonance imaging (MRI) or magnetic resonance spectroscopy (MRS) and thereby determining one or several pH values or pH changes of or in the body of said patient or non-human animal by obtaining the chemical shift difference between at least one pH sensitive chemical shift of the compound and a pH-independent chemical shift, such pH-independent chemical shift acting as a reference chemical shift, or by measurement of the absolute chemical shift, or by measuring chemical shift differences involving at least one pH-sensitive chemical shift over time,
(iii) calculating pH maps based on spatially resolved pH values or pH changes determined in step (ii).

Step (iii) can comprise
comparing said relative chemical shifts to predetermined calibration curves of the
compound with at least one pH-sensitive chemical shift in solutions with known pH. The method further can comprise
hyperpolarizing the compound with at least one pH-sensitive chemical shift before
application or administration to the body of the patient.

Thereby, the compound is hyperpolarized by any hyperpolarization methods, such as dissolution dynamic nuclear polarization (DNP) or parahydrogen induced polarisation (PHIP). "A disease causing changes in pH" can be selected from cancers, inflammation, ischemia, renal failure and chronic obstructive pulmonary disease.

Thereby, the progress of a disease and/or the treatment of a disease can be monitored.

The method can comprise magnetic resonance tomography (MRT).

The imaging can be real-time.

The method can comprise the resolution of the spatial pH distribution and, thus, obtaining spatially resolved NMR spectra,
such as, comprising the use of frequency encoding techniques, such as all methods of chemical shift imaging (CSI) and phase sensitive encodings of chemical shifts (as e.g. used in non-invasive spatially resolved temperature measurements using changes in proton resonance frequencies (0.01 ppm/°C)). See Rieke et al., 2004.

### Further uses

Further described is the use of a compound of the present disclosure or a biosensor described in the present disclosure as in quality control of food or in the examination of plants and organisms.

### Further description of preferred disclosure items

Local changes of pH in the human body are triggered by many pathologies that overrule natural pH regulatory mechanisms, in particular tumors, inflammation, and ischemia, but also renal failure and chronic obstructive pulmonary disease. The spatially resolved, robust, and non-invasive method for the exact measurement of local pH and its means, described herein, therefore offer improved means for preclinical and clinical applications both for diagnostics and therapeutical purposes, such as monitoring response-to-treatment. Furthermore, applications range from quality control of food to the examination of plants and organisms.

Here, a novel pH biosensor is presented (that is based on compounds with at least one pH-sensitive chemical shift, such as zymonic acid or its analogs or OMPD or its analogs) for magnetic resonance that is very sensitive to pH-changes in a physiologically and/or pathologically relevant pH range. This novel sensor acts independently of its concentration and enzymatic reactions and therefore allows a very accurate pH mapping at high spatial resolution making it a promising probe for the translation to the clinic.

The invention is based on the fact that the chemical compound zymonic acid exhibits a pH-dependent chemical shift for some of its ¹³C- and ¹H-resonances (marked in Figure 1).

Zymonic acid has been discovered and named in the 1950s for the first time and can be produced by yeast bacteria from glucose or can originate from a ring closure of parapyruvate molecules which in turn can originate from pyruvic acid (Bloomer et al., 1970 -1; Bloomer et al., 1970 -2; Stodola et al., 1952). Zymonic acid is used as a flavor constituent for confectionary and in the tobacco industry and is therefore most likely not toxic when administered *in vivo.*

The invention is further based on the fact that the chemical compound OMPD ((*Z*)-4-methyl-2-oxopent-3-enedioic acid), which can be produced from pyruvate through catalysis by strong acid, exhibits a pH-dependent chemical shift for its ¹³C- and ¹H-resonances (see Figure 7). OMPD was first discovered in tulip plants in 1988 (Ohyama et al., 1988-1; Ohyama et al., 1988-2; Ohyama et al., 2006).

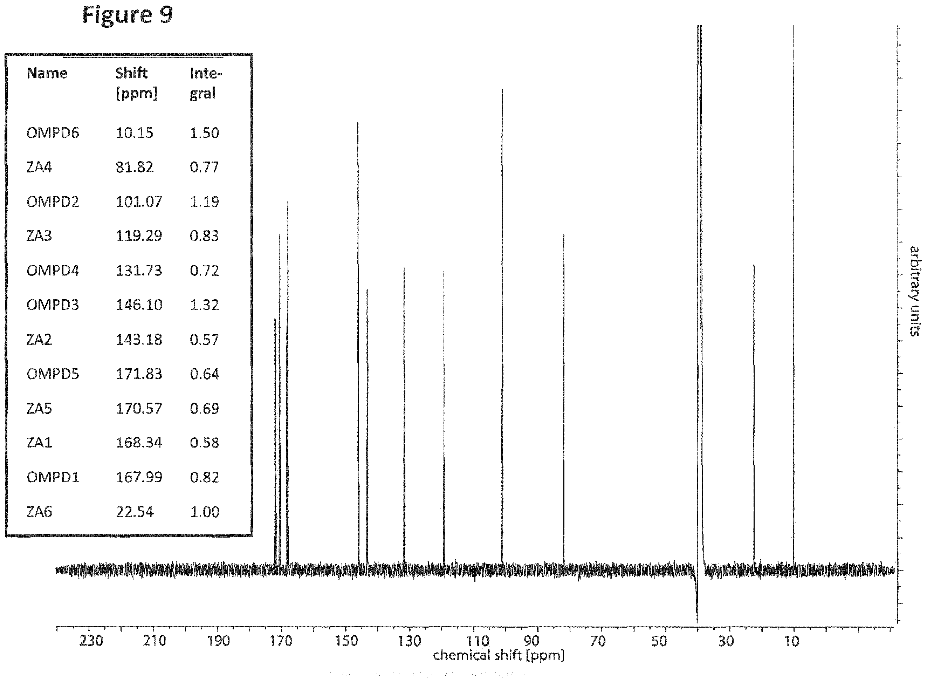

Both, zymonic acid and OMPD are extremely sensitive pH biosensors for magnetic resonance spectroscopy and magnetic resonance tomography and exhibits the following properties, which sets zymonic acid apart from other non-invasive methods to measure pH:
(a) Zymonic acid and OMPD exhibit the highest pH-dependent changes in chemical shift measured to date with ¹³C shifts up to 2.35 ppm/pH (zymonic acid) or 12.2 ppm (OMPD), respectively, in the physiologically and/or pathologically relevant range from pH 5 to pH 9 and are thus suitable for a very accurate noninvasive pH determination using magnetic resonance tomography/spectroscopy.
(b) Unlike some other pH measurement methods, the pH determination is robustly performed using a relative or absolute frequency encoding as long as a pH independent reference, e.g. urea or a pH independent chemical shift of zymonic acid/OMPD itself, is present in the sample. In contrast, the amplitude encoding used in the bicarbonate method is prone to spatial or temporal fluctuations in concentration.
(c) ¹³C-labeled zymonic acid (see chemical shifts 1 and 2 in Figure 6) and ¹³C-labeled OMPD (see Figure 9) can be produced from pyruvate or pyruvic acid (either ¹³C-labeled or not, such as [1-¹³C]-pyruvate) in a one-step synthesis.

The two carboxyl/ester groups of both compounds are exposed to a weak dipolar interaction and thus exhibit a long longitudinal relaxation time T₁. Hyperpolarization increases the polarization of the molecule by a factor 50,000 which enables pH imaging in the human body at low contrast agent concentration and at the same time high spatial resolution.

Medical applications for pH imaging with these new pH sensors are extremely numerous since many pathologies cause changes in pH. Good examples are tumors, inflammation and ischemia, but also renal failure and chronic obstructive pulmonary disease. Furthermore, the application as a very precise pH sensor for *in vitro* NMR-spectroscopy is interesting, e.g. for response-to-treatment monitoring of treatments applied to cell lines.

The pH-dependent change in chemical shifts of zymonic acid and/or OMPD can be used in magnetic resonance tomography to resolve the spatial pH distribution for which established frequency encoding techniques can be used. This includes all spectrally resolving variations of chemical shift imaging (CSI) as well as phase sensitive encodings of chemical shifts as e.g. used in non-invasive spatially resolved temperature measurements using changes in proton resonance frequencies (0.01 ppm/°C).

The following examples and drawings illustrate the present invention.

### BRIEF DESCRIPTION OF THE DRAWINGS

**Figure 1****.**
   (A) *Schematic depiction of zymonic acid.*
      Shown are zymonic acid's pKa-values assigned to the relevant proton donating groups. Two exemplary ¹³C resonances, which show a pH-dependent change (or displacement) in chemical shift, are marked in light grey and numbered 1 and 2 according to their increasing ¹³C-NMR resonance frequency (the pH-dependent proton resonances are marked in darker grey and numbered A and B according to their increasing ¹H-NMR resonance frequency). It should be noted that the two ¹³C resonances shown here are examples of pH-sensitive chemical shifts; however, also the other carbon atoms in zymonic acid, if ¹³C-labelled for increased sensitivity, will show such pH-sensitive chemical shifts.
   (B) Schematic depiction of pKa value
      Figure 1B) shows the protonated and unprotonated forms of compounds such as zymonic acid. Shown on the left is the protonated form, and on the right the deprotonated form.
   (C) Deprotonated conformation of zymonic acid.
**Figure 2****.**
   **(A)** pH dependent ¹³C chemical shifts from peaks 1 and 2 in ¹³C-NMR spectra of zymonic acid.
   **(B)** pH dependent ¹³C chemical shifts from peaks 1 and 2 in ¹³C-NMR spectra of zymonic acid and best fit straight lines. Peak 1 of zymonic acid shows a pH dependent change in chemical shift of approx. 2.1 ppm/pH, and peak 2 of approx. 1.1ppm/pH. The error bars are calculated from the pH values of the sample determined with a standard pH electrode before and after the NMR-measurement. The spectrum was referenced to ¹³C-urea at 0 ppm.

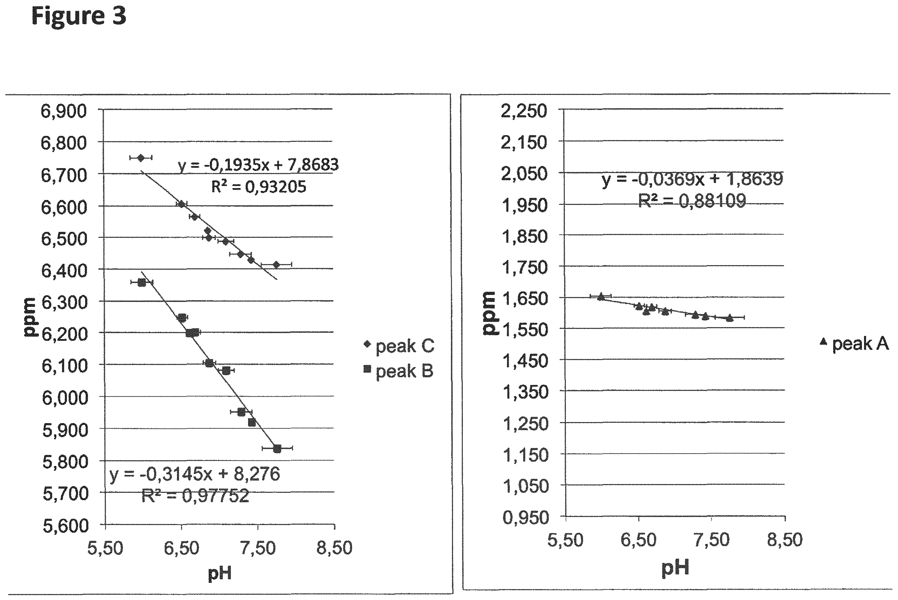

**Figure 3****.** *Proton chemical shifts of the proton peaks 4 and 5 of zymonic acid as a function of pH value.*
   **(A)** and **(B):** The additional peak C is produced during the synthesis of zymonic acid from pyruvate and can be assigned to OMPD. The pH dependent change in chemical shift of peak B of zymonic acid is approx. -0.3 ppm/pH and peak A approx. -0.04 ppm/pH. Again, the error bars are calculated from the pH values of the sample determined with a standard pH electrode before and after the NMR-measurement. The spectrum was referenced to the external standard TMS at 0 ppm.
**Figure 4****.**
   Injection of hyperpolarized [1-¹³C]-pyruvate in 40x10⁶ MCF-7 tumor cells in two series of measurements **(A, B)**, in which the tumor cells were killed by addition of Triton-X100, which led to a gradually increasing acidification of the medium. The pH value was determined with a standard pH electrode after the NMR-measurement. The ¹³C spectra were referenced to the [1-¹³C]-pyruvate peak at 6.5 ppm. **(C)** Two peaks, which can be assigned to the hyperpolarized zymonic acid, show a strong pH dependent linear change in chemical shift with up to 2.35 ppm/pH.

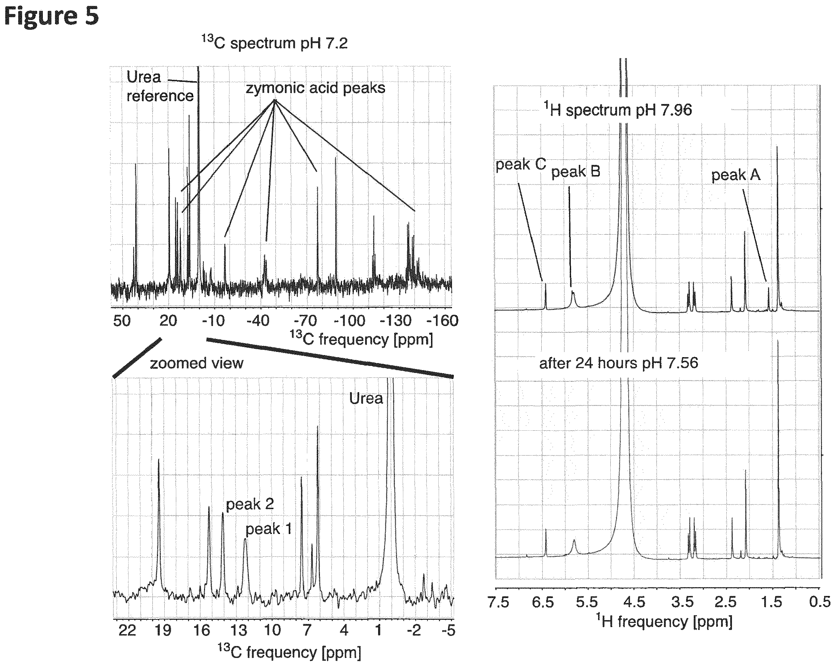

**Figure 5****.** *¹³**C spectrum and ¹H spectrum of zymonic acid produced from [1-¹³C]-pyruvic acid.*
   An exemplary ¹³C spectrum (left column) and ¹H spectrum (right column) of zymonic acid produced from [1-¹³C]-pyruvic acid. The relevant peaks 1 to 5, which shift in dependency of the pH value of the sample, are marked. The proton spectra show a buffered solution of zymonic acid in water and D₂O, once measured right after preparation of the solution (top right) and 24h later (bottom right).
**Figure 6****.** *Confirmation of the structure of zymonic acid using mass spectrometry.*
   **(A)** The HR-MS-spectrum of the synthesized substance recorded with a Thermo Finnigan LTQ-FT confirms the total mass of the compound.
   **(B)** The MS/MS-spectrum of the synthesized substance recorded after CID-fragmentation on a Thermo Finigan LCQ-Fleet and the assignment of the observed fragments confirms the assumed structure.

   Within the accuracy of the ion trap (±0.3 m/z), all peaks can be explained by elimination of carbon monoxide and carbon dioxide.
**Figure 7****.** *Schematic depiction of OMPD.*
   The numbered ¹³C resonances show a pH-dependent change in chemical shift. Also the proton resonances show a pH-dependent change in chemical shift.
**Figure 8****.**
   Chemical shifts in Δppm of all six carbon atoms in OMPD relative to their chemical shift at pH 7.26 as a function of pH. The pH of each sample was determined with a standard pH electrode before and after the NMR-measurement. Shown is the average pH of both measurements.
**Figure 9****.** *¹³**C spectrum of OMPD and zymonic acid produced from pyruvic acid.*
   An exemplary ¹³C spectrum of OMPD and zymonic acid (ZA) produced from pyruvic acid dissolved in DMSO.
**Figure 10****.** *¹³C-spectra of OMPD for measurement of extracellular tumor cell pH.*
   Ten ¹³C-spectra (proton decoupled) of ca. 6 mM fully-labeled ¹³C-OMPD dissolved in cell culture medium with ca. 10 Mio. MCF-7 tumor cells immobilized in alginate beads were acquired during a total time of 10 hours within every hour. OMPD was not metabolized within tumor cells. pH-sensitive OMPD-peaks 1, 5, and 6 showed small pH-dependent shifts due to acidification.

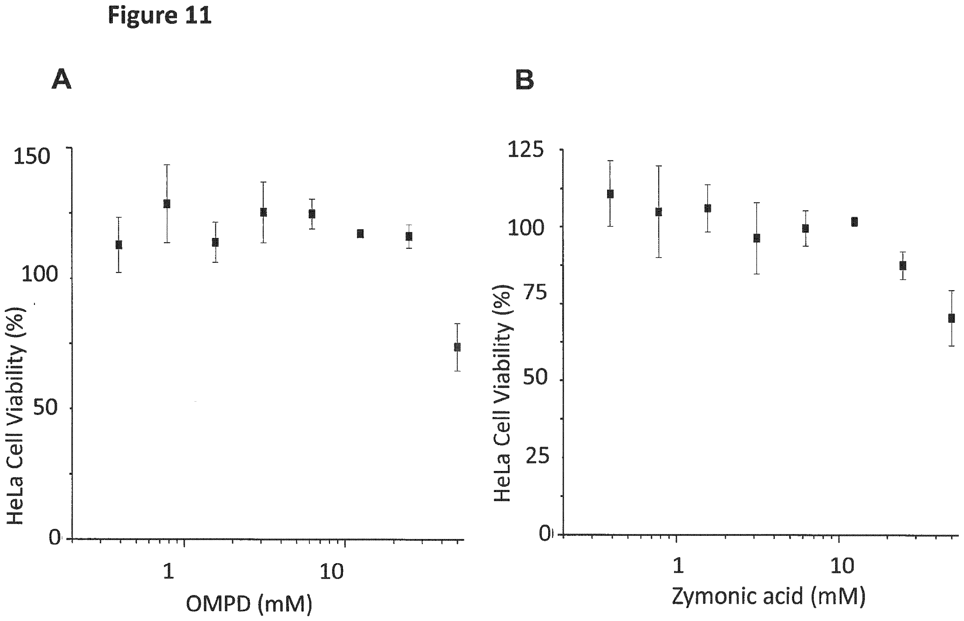

**Figure 11****.** *Zytotox-test of purified zymonic acid and OMPD*
   MTT assay with HeLa cells for testing cell viability after exposure to (A) OMPD and (B) zymonic acid (ZA) (0.4 to 50 mM) for 24 hours.
   (12C, ZA: 100% pure; OMPD: 80% OMPD, 20% ZA).
   After 24 hours incubation time, both substances are not cytotoxic up to the tested concentration of 50 mM.

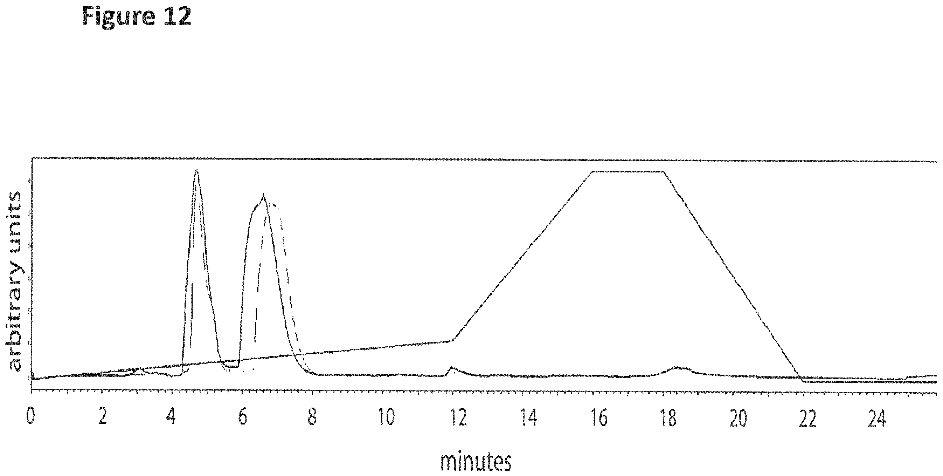

**Figure 12****.** *Separation of zymonic acid and OMPD using HPLC*
   HPLC chromatograms of the residue that was obtained from treating pyruvic acid with conc. hydrochloric acid and subsequent evaporation of all volatiles *in vacuo.*
   Separation was achieved with a Waters 2545 quaternary gradient module, an X-Bridge™ Prep C18 10 µm OBD™ (50 x 250 mm), a Waters 2998 PDA detector and a Waters Fraction Collector III (detection at 210 nm). A gradient from 2% to 20% B was run over 12 min, where solvent A consisted of 0.1% (v/v) TFA in water and solvent B consisted of 0.1% (v/v) TFA in acetonitrile. The peak at 4.8 min consisted of 50% - 100% of OMPD and 0 - 50% of zymonic acid (average values of over 10 experiments). The peak at around 6.8 min consisted of zymonic acid.

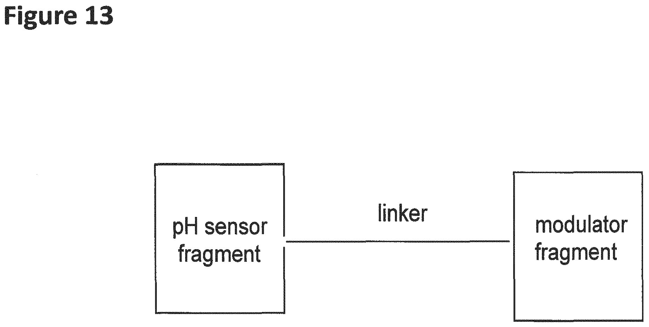

**Figure 13****.** *A modular biosensor of the invention.*

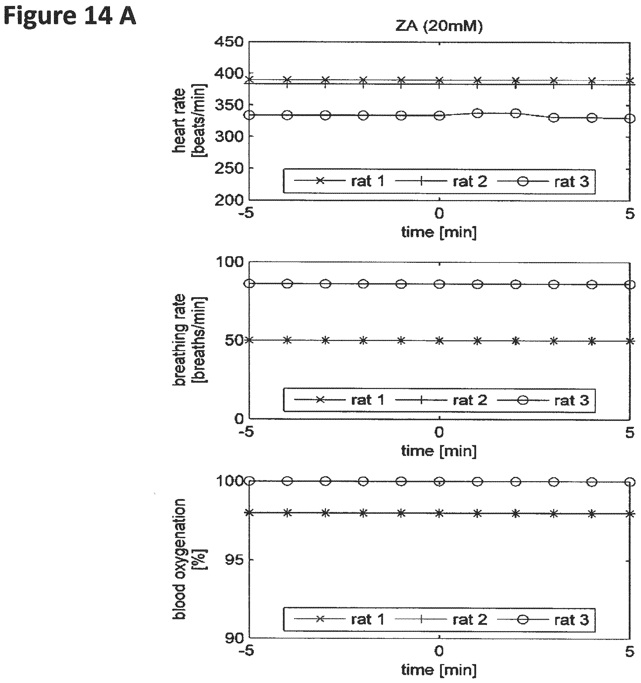

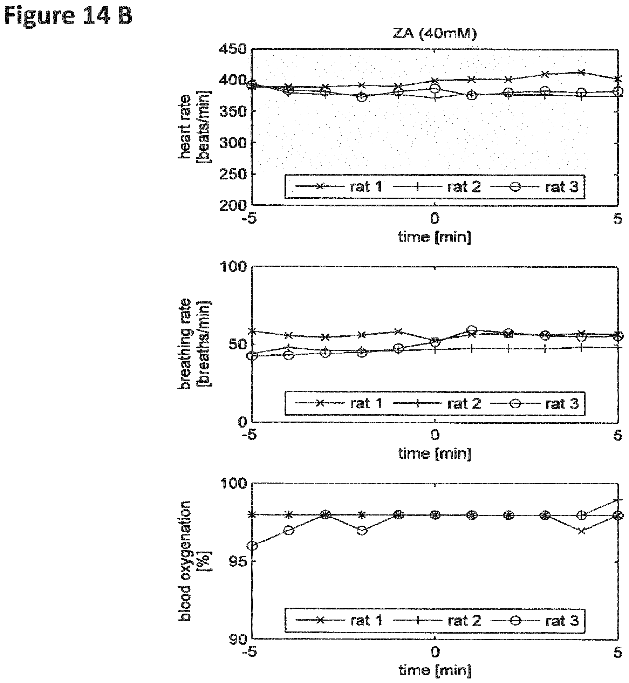

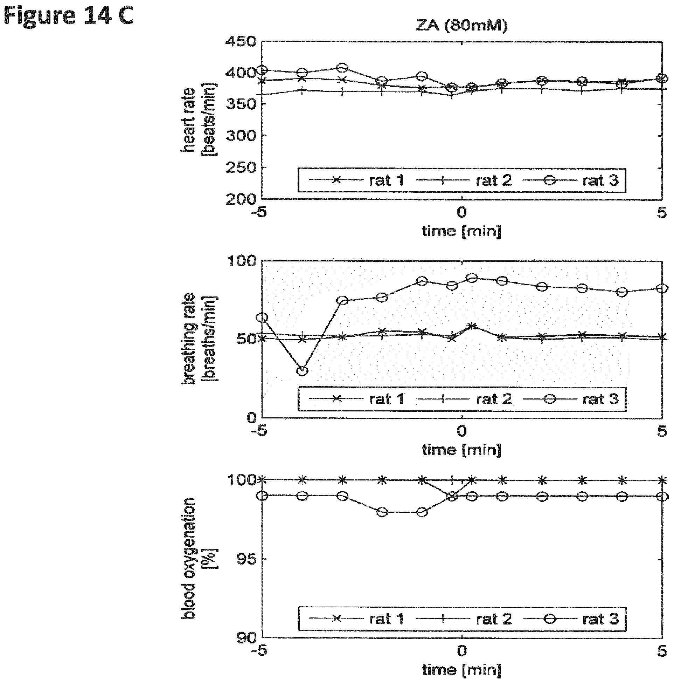

**Figure 14****.** *Toxicity studies of zymonic acid in rats.*
   Toxicity tests with a volume of 5mL/kg at an injection rate of 0.2 mL/s for three different concentrations (20 mM **(A)**, 40mM **(B)**, 80 mM **(C)**). Injection solution contained 80 mM Tris buffer solution, titrated with 1M NaOH to pH 7.4.

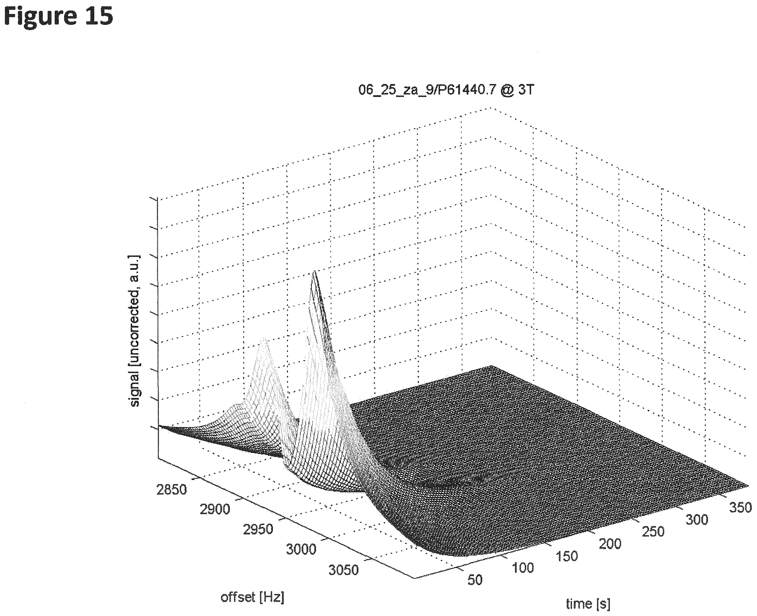

**Figure 15****.** *Decay of hyperpolarized zymonic acid at 3T.*

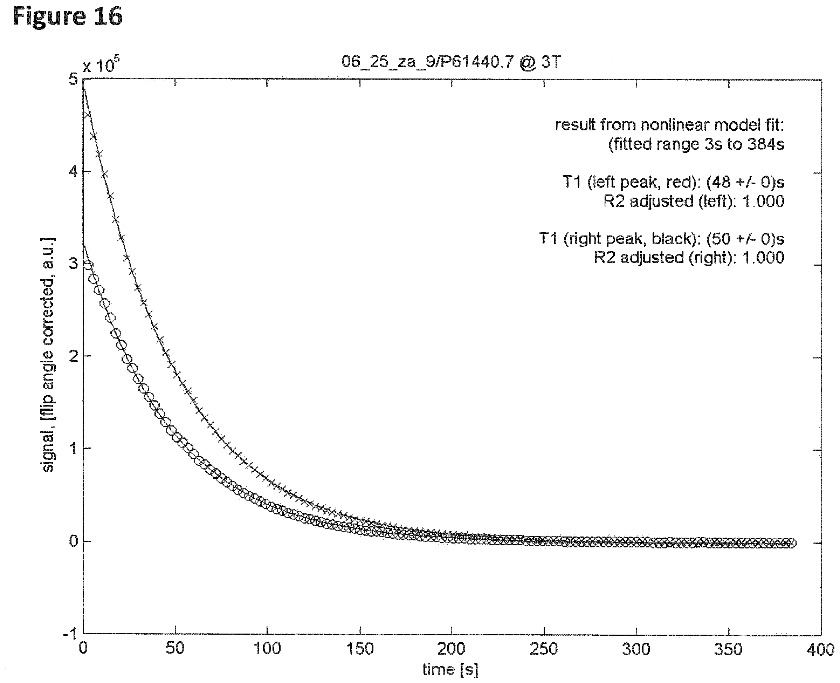

**Figure 16****.** *Decay of hyperpolarized zymonic acid (position 1 and 5) at 3T.*
   Maximum peak intensities are shown.

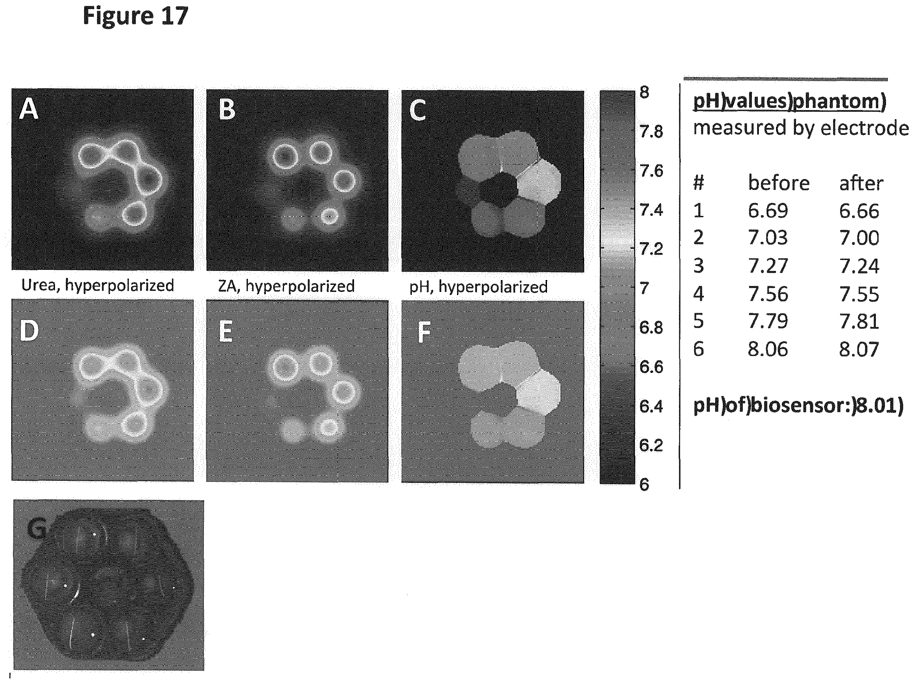

**Figure 17****.** *Copolarization of zymonic acid and urea and injection into Eppendorf tubes at different pH values.*
   Imaging parameters were: sequence = FIDCSI, field of view = 6 cm x 6 cm, matrix: 16 x 16, excitations = 210, flip angle = 3°, TR = 118 ms, total scan time = 25s, phantoms were adjusted to contain: 6 x 2mL 80 mM TRIS buffer with pH 6.7, 7.0, 7.3, 7.6, 7.8, and 8.1. The pH sensor consisted of 6 batches of 200uL copolarized Urea and ZA in 20 mM TRIS buffer and NaOH.
   The center syringe consists of 1M urea (not hyperpolarized). (A) Urea image, (B) ZA image, (C) calculated pH image, (D) overlay of proton image with Urea image, (E) overlay of ZA image with proton image, (F) overlay of pH image with proton image, (G) photograph of the phantom.

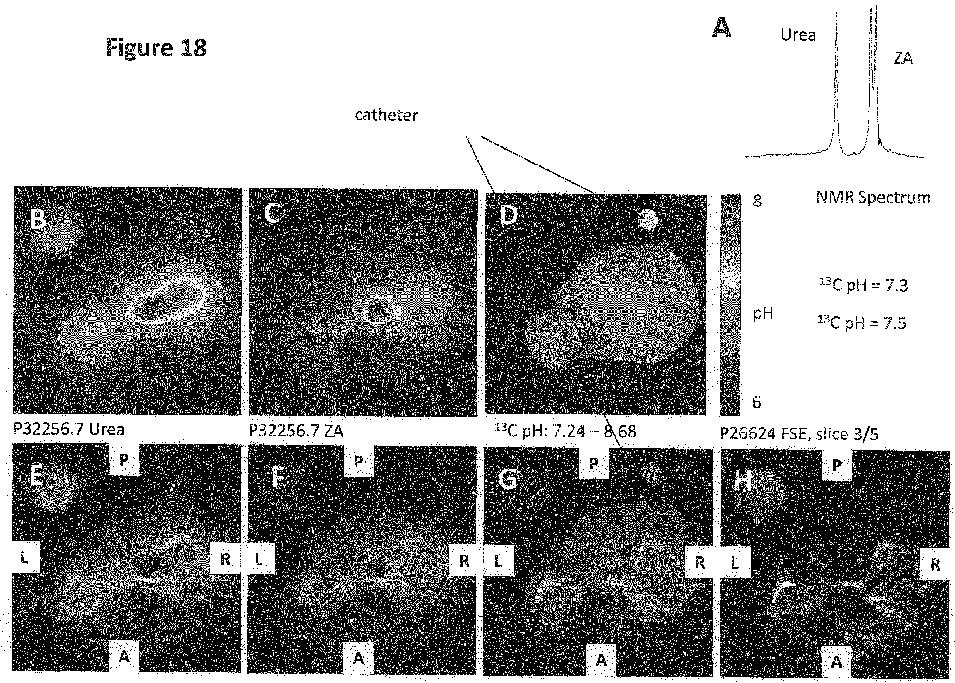

**Figure 18****.** *Copolarization of zymonic acid and urea, injection into a healthy rat via the tail vein.*
   A 10 mm slice covering the kidney volume was chosen. (A) Summed spectra over entire slice, (B) Urea image, (C) ZA image, (D) calculated pH image, (E) overlay of proton image with Urea image, (F) overlay of ZA image with proton image, (G) overlay of pH image with proton image, (H) Fast spin echo proton image.

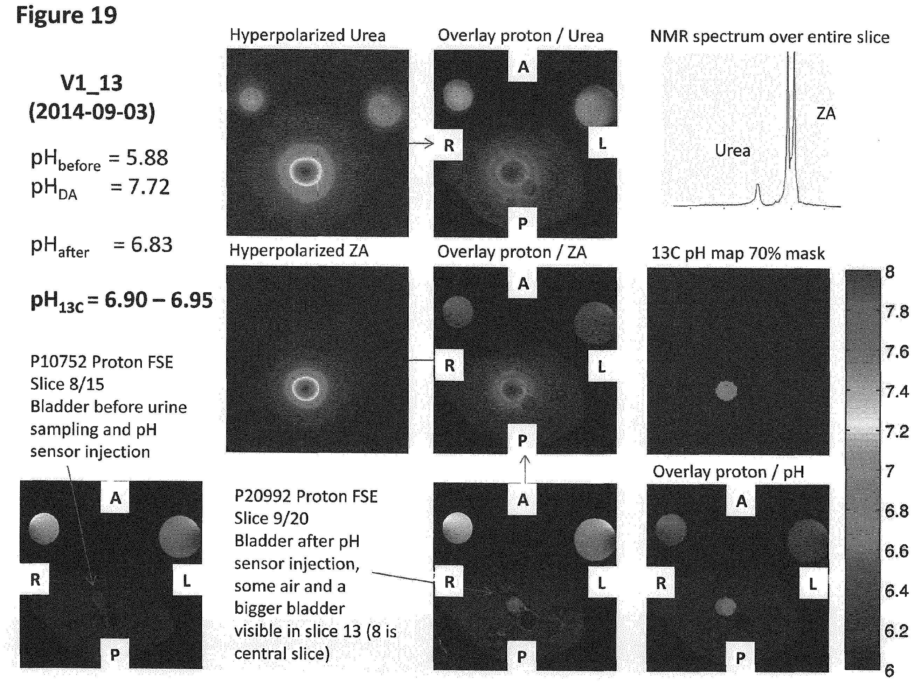

**Figure 19****.** *Copolarization of zymonic acid and urea, injection into the rat bladder via a catheter.*
   A 10 mm slice covering the bladder volume was chosen. Electrode pH measurements confirm the pH values measured by NMR spectroscopy using hyperpolarized ZA.

### EXAMPLES

### EXAMPLE 1

### Materials and Methods

All experiments with zymonic acid described herein were conducted in the Department of Chemistry at Technische Universität Munchen on a Bruker® 14.1 T NMR spectrometer with an AVANCE III console.

pH values were measured with a standard pH electrode.

### Production and characterization of zymonic acid

Zymonic acid was produced from pyruvic acid as described in the literature (De Jong, 1901). To this end, concentrated hydrochloric acid was added to pyruvic acid in a 1:1 volume ratio. The reaction mixture was then allowed to stand for two weeks at room temperature. Volatile compounds were removed *in vacuo* whereupon the yellow oil obtained showed crystallization. The yellow and strongly hygroscopic solid was then used without further purification.

The formula of zymonic acid is shown in Figure 1 (showing its pKa-values (Montgomery et al., 1954) assigned to the relevant proton donating groups and the pH-dependent ¹³C resonances and pH-dependent proton resonances).

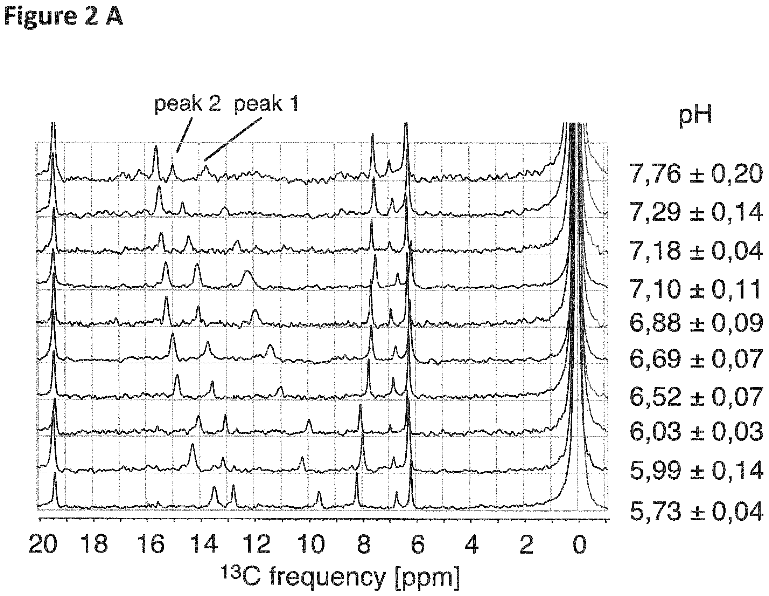

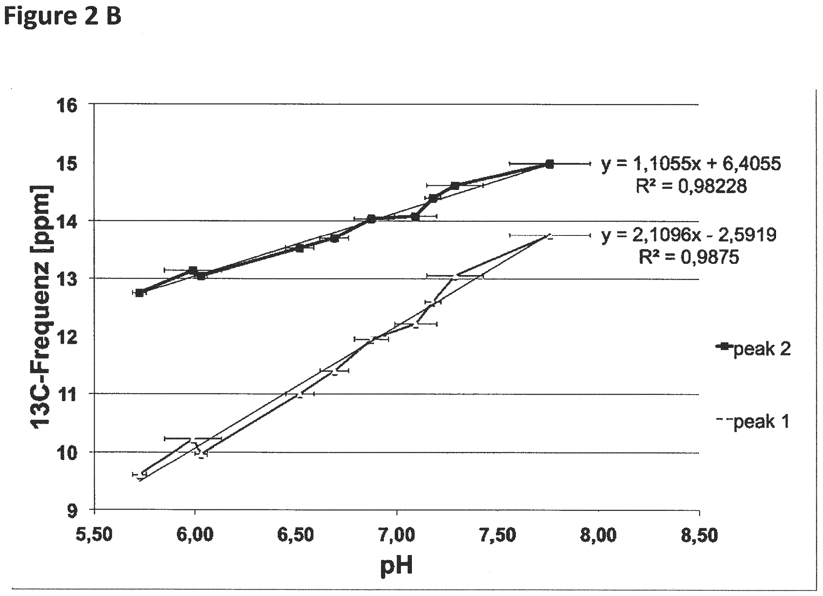

For the measurement of pH, calibration curves in 200 - 500 mM aqueous solutions of the reaction product were used in sodium phosphate buffer (1M). The pH was then adjusted by cautious addition of sodium hydroxide solution (10 M) or concentrated hydrochloric acid. 5-15% (v/v) D₂O and ¹³C-urea were added for referencing. The pH-dependent chemical shift of the relevant NMR peaks of zymonic acid is depicted in Figure 2. We did not observe hysteresis effects in the pH-dependent shift. In particular, Figure 2 shows the pH dependent ¹³C chemical shifts from peaks 1, 2 and 3 in ¹³C-NMR spectra of zymonic acid and their best fit straight lines. Peak 1 of zymonic acid shows a pH dependent change in chemical shift of approx. 2.1 ppm/pH, peak 2 of approx. 1.1ppm/pH and peak 3 of approx. 1.0 ppm/pH.

The assignment of the NMR-peaks of zymonic acid was done using NMR prediction software (ChemDraw®) and standard 1D- and 2D-NMR-spectroscopy (see Figure 5). In particular, Figure 5 shows an exemplary ¹³C spectrum (left column of Figure 5) and ¹H spectrum (right column of Figure 5) of zymonic acid produced from [1-¹³C]-pyruvic acid. The relevant peaks 1 to 5, which shift in dependency of the pH value of the sample, are marked. Since zymonic acid slowly decomposed into parapyruvate within a period of 24 hours (Montgomery et al., 1956), its peaks can be assigned from subtraction of one spectrum from the other in combination with standard NMR prediction software (ChemDraw®). The proton spectra show a buffered solution of zymonic acid in water and D₂O, once measured right after preparation of the solution (top right of Figure 5) and 24h later (bottom right of Figure 5).

Mass spectrometry confirmed the chemical formula of zymonic acid (see Figure 6). Thereby, Figure 6A shows the HR-MS-spectrum of the synthesized substance which confirms the total mass of the compound. Figure 6B shows the MS/MS-spectrum of the synthesized substance recorded after CID-fragmentation, wherein the assignment of the observed fragments confirms the assumed structure. Within the accuracy of the ion trap (±0.3 m/z), all peaks can be explained by elimination of carbon monoxide and carbon dioxide.

### Detection of tumor cell death due to pH change

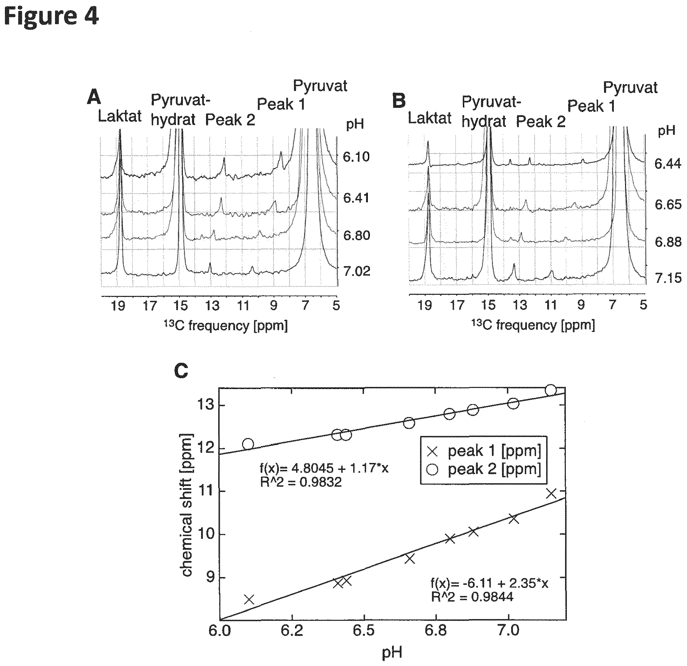

The pH dependent peaks 1 and 2 of zymonic acid were also observed in ¹³C-NMR spectra of hyperpolarized, ¹³C-labeled [1-¹³C]-pyruvate in MCF-7 tumor cells (see Figure 4).

Zymonic acid is formed from parapyruvate by a ring closure. In this process, the originally ¹³C-labeled carboxyl groups create a ¹³C-labeling of zymonic acid in positions 1 and 2 (see Figure 1). The pH dependent chemical shift of peak 1 of zymonic acid was determined to be approx. 2.35 ppm/pH in this measurement and approx. 1.17 ppm/pH for peak 2, which is in good agreement with the results from the thermally polarized and unlabeled zymonic acid (cf. Figure 2, i.e. 2.11 ppm/H and 1.11 ppm/pH, respectively).

The tumor cells were treated with Triton X-100 so that they gradually become necrotic with time and that pH decreases successively, similar to the case of a necrotic tumor. As an exemplary application, this pH change in tumor cells can be detected using our pH biosensor. The pH of the tumor cell suspension was determined immediately after the NMR-measurement using a pH electrode as a reference (see Figure 4A, B). As is demonstrated in Figure 4C, two peaks, which can be assigned to the hyperpolarized zymonic acid, show a strong pH dependent linear change in chemical shift with up to 2.35 ppm/pH.

### EXAMPLE 2

### Materials and Methods

All experiments with OMPD described herein were conducted in the Department of Chemistry at Technische Universität München on a Bruker® 14.1 T NMR spectrometer with an AVANCE III console.

pH values were measured with a standard pH electrode.

### Production and characterization of OMPD

OMPD was produced as a byproduct from the synthesis of zymonic acid from pyruvic acid (De Jong, 1901). To this end, this is the first time that this reaction is described. Concentrated hydrochloric acid was added to pyruvic acid in a 1:1 volume ratio. The reaction mixture was then allowed to stand over concentrated sulfuric acid in a dessicator for two weeks at room temperature. Volatile compounds were removed *in vacuo* whereupon the yellow oil obtained showed crystallization. The yellow and strongly hygroscopic solid containing both OMPD and zymonic acid was then used without further purification. Separation of OMPD and zymonic acid was achieved my means of high-pressure liquid chromatography.

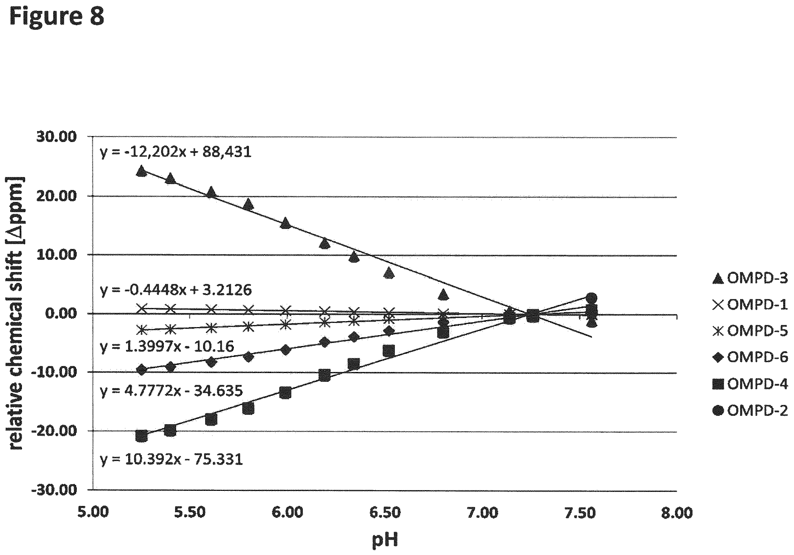

The assignment of the NMR peaks of OMPD (see Figure 9) is given in Table 1 and was done using NMR prediction software (ChemDraw®), comparing the obtained chemical shift values to the ones reported in literature¹³⁻¹⁵, and standard ID- and 2D-NMR-spectroscopy (see Figure 8). OMPD and zymonic acid have equivalent molecular weights.

**Table 1. ¹H- and ¹³C-NMR data on OMPD in DMSO.**

| Values of the chemical shifts were obtained setting the DMSO multiplet to 39.52 ppm for ¹³C-NMR and 2.50 ppm for ¹H-NMR data. *J*-couplings were determined via automated multiplet analysis (Mnova®). Multiplets are described as (s) singlet, (d) doublet, (q) quartet. | | | |
|---|---|---|---|
| Position | | C | H |
| OMPD-1 | 168.2 (s) | | |
| OMPD-2 | 101.1 (d) | ²J_{C2-H3} = 7.5 Hz | |
| OMPD-3 | 146.1 (dq) | ¹J_{C3-H3} = 179.5 Hz | 7.22 (1H, q, J=1.6Hz) |
| | | ³J_{C3-H6} = 5.4 Hz | |
| OMPD-4 | 131.8 (dq) | ²J_{C4-H3} = 7.2 Hz | |
| | | ² J_{C4-H6} = 3.5 Hz | |
| OMPD-5 | 171.9 (dq) | ³J_{C5-H3} = 13.2 Hz | |
| | | ³J_{C5-H6} = 4.3 Hz | |
| OMPD-6 | 10.2 (qd) | ¹J_{C6-H6} = 129.1 Hz | 1.84 (3H, d, J=1.6Hz) |
| | | ³J_{C6-H3} = 2.8 Hz | |

In order to determine the pH-dependent chemical shifts, calibration curves in 200 - 500 mM aqueous solutions of the reaction product were used in sodium phosphate buffer (1M). The pH was then adjusted by cautious addition of sodium hydroxide solution or concentrated hydrochloric acid. 5-15% (v/v) D₂O and ¹³C-urea were added for reference. The pH-dependent chemical shifts of the relevant NMR peaks of OMPD acid are depicted in Figure 8. pH-values were measured in random order and we did not observe hysteresis effects in the pH-dependent chemical shift.

### Measurement of extracellular tumor cell pH

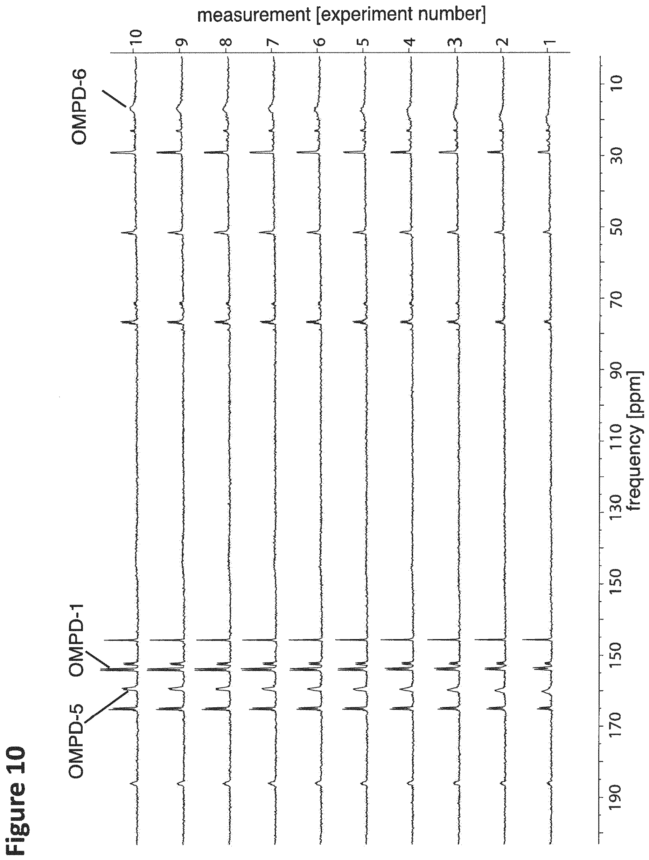

The pH sensitive OMPD-peaks 1, 5 and 6 of zymonic acid were also observed in ¹³C-NMR spectra of ¹³C-OMPD dissolved in cell culture medium with MCF-7 tumor cells immobilized in alginate beads. OMPD was not metabolized within tumor cells. pH-sensitive OMPD-peaks 1, 5, and 6 showed small pH-dependent shifts due to acidification. See Figure 10.

### EXAMPLE 3

### Cytotoxicity test of zymonic acid and OMPD

Purified zymonic acid and OMPD were tested in a MTT assay with HeLa cells for testing cell viability after exposure to each substance at concentrations of 0.4 to 50 mM for 24 hours. After 24 hours incubation time, both substances are not cytotoxic up to the tested concentration of 50 mM. See Figure 11.

### EXAMPLE 4

[1,5-¹³C]ZA can be synthetized with ca. 35 % yield from [1-¹³C] pyruvic acid (duration 4 days). Structure simulations determine the conformation and let us understand the mechanism of ZA's pH sensitivity (see Figure 1C).

### Toxicity tests in rats

20 mM, 40 mM, and 80 mM zymonic acid were injected into 3 rats within one week with 1 day break in between each injection (see Figure 14).

Rats survived all injections and did not show health problems even after 3 days post last injection. Blood pressure, heart rate, blood oxygenation and animal temperature were monitored 10 minutes before and after the injection. No unusual behaviour of the animals was observed.

### Hyperpolarization of zymonic acid

Zymonic acid was successfully hyperpolarized (T₁ @ 3T ca. 40-50s, T₁ @ 7T ca. 20s) (see Figure 15 and 16).

Co-polarization of zymonic acid with ¹³C urea was established (see Figure 17).

### In vitro experiments

10x test experiments and phantom experiments with varying pH (see Figure 17).

20x polarization and co-polarization tests.

### In vivo pH determination in rat kidneys and bladders

2 injections of copolarized zymonic acid and urea in healthy rats via the tail-vein followed by kidney pH-imaging (see Figure 18).

6 injections of copolarized zymonic acid in healthy rats via a catheter directly into the bladder followed by pH-imaging and pH-control of the urine before an after injection by pH-electrode (see Figure 19).

### REFERENCES

Aime S, Delli Castelli D, Terreno E. Novel pH-reporter MRI contrast agents. Angew Chem Int Ed Engl 2002;41:4334-6.
Ardenkjaer-Larsen JH, Fridlund B, Gram A, et al. Increase in signal-to-noise ratio of > 10,000 times in liquid-state NMR. Proceedings of the National Academy of Sciences of the United States of America 2003;100:10158-63.
Arnold DL, Matthews PM, Radda GK. Metabolic recovery after exercise and the assessment of mitochondrial function in vivo in human skeletal muscle by means of 31P NMR. Magnetic resonance in medicine : official journal of the Society of Magnetic Resonance in Medicine / Society of Magnetic Resonance in Medicine 1984;1:307-15.
Bloomer JL, Gross MA. Biosynthesis of Zymonic Acid in Trichosporon-Capitatum. J Chem Soc Chem Comm 1970:73-74.
Bloomer JL, Gross MA, Kappler FE, Pandey GN. Identity of Zymonic Acid with a Pyruvate Derivative. J Chem Soc Chem Comm 1970:1030.
Castelli DC, Ferrauto G, Cutrin JC, Terreno E, Aime S. In vivo maps of extracellular pH in murine melanoma by CEST-MRI. Magnetic resonance in medicine : official journal of the Society of Magnetic Resonance in Medicine / Society of Magnetic Resonance in Medicine 2013: 1-8.
Day SE, Kettunen MI, Gallagher FA, Hu DE, Lerche M, Wolber J, Golman K, Ardenkjaer-Larsen JH, Brindle KM. Detecting tumor response to treatment using hyperpolarized 13C magnetic resonance imaging and spectroscopy. Nat Med. 2007 Nov;13(11):1382-7.
De Graaf, Robin. In Vivo NMR Spectroscopy: Principles and Techniques, John Wiley & Sons; 2007.
De Jong MAKW. L'action de l'acid chlorhydrique sur l'acide pyruvique. Recueil des travaux chimiques des Pays-Bas 1901;20:81-101.
De Leon-Rodriguez LM, Lubag AJ, Malloy CR, Martinez GV, Gillies RJ, Sherry AD. Responsive MRI agents for sensing metabolism in vivo. Accounts of chemical research 2009;42:948-57.
Ernst, Richard. Principles of Nuclear Magnetic Resonance in One and Two Dimension (International Series of Monographs on Chemistry), Oxford University Press; 1997.
Gallagher FA, Kettunen MI, Day SE, Hu DE, Ardenkjaer-Larsen JH, Zandt Ri, Jensen PR, Karlsson M, Golman K, Lerche MH, Brindle KM. Magnetic resonance imaging of pH in vivo using hyperpolarized 13C-labelled bicarbonate. Nature. 2008 Jun 12;453(7197):940-3.
Gillies RJ, Raghunand N, Garcia-Martin ML, Gatenby RA. pH imaging. A review of pH measurement methods and applications in cancers. IEEE engineering in medicine and biology magazine : the quarterly magazine of the Engineering in Medicine & Biology Society 2004;23:57-64.
Hassan M, Riley J, Chernomordk V, et al. Fluorescence lifetime Imaging system for in vivo studies. Mol Imaging 2007;6:229-36.
Jensen PR, Karlsson M, Lerche MH, Meier S. Real-time DNP NMR observations of acetic acid uptake, intracellular acidification, and of consequences for glycolysis and alcoholic fermentation in yeast. Chemistry. 2013; 19(40):13288-93.
Montgomery CM, Webb JL. Detection of a New Inhibitor of the Tricarboxylic Acid Cycle. Science 1954;120:843-4.
Montgomery CM, Webb JL. Metabolic studies on heart mitochondria. II. The inhibitory action of parapyruvate on the tricarboxylic acid cycle. The Journal of biological chemistry 1956;221:359-68.
Morikawa S, Inubushi T, Kito K, Kido C. pH mapping in living tissues: an application of in vivo 31P NMR chemical shift imaging. Magnetic resonance in medicine : official journal of the Society of Magnetic Resonance in Medicine / Society of Magnetic Resonance in Medicine 1993;29:249-51.
Nelson SJ, Ozhinsky E, Li Y, Park I, Crane J. Strategies for rapid in vivo 1H and hyperpolarized 13C MR spectroscopic imaging. J Magn Reson 2013;229:187-97.
Nelson SJ, Kurhanewicz J, Vigneron DB, Larson PE, Harzstark AL, Ferrone M, van Criekinge M, Chang JW, Bok R, Park I, Reed G, Carvajal L, Small EJ, Munster P, Weinberg VK, Ardenkjaer-Larsen JH, Chen AP, Hurd RE, Odegardstuen LI, Robb FJ, Tropp J, Murray JA. Metabolic Imaging of Patients with Prostate Cancer Using Hyperpolarized [1-13C]Pyruvate. Sci Transl Med. 2013 Aug 14;5(198):198ra108.
Ohyama T, Hoshino T, Ikarashi T. Isolation and Structure of a New Organic-Acid Accumulated in Tulip Plant (Tulipa-Gesneriana). Soil Sci Plant Nutr 1988;34:75-86.
Ohyama T, Kera T, Ikarashi T. Occurrence of 4-Methyleneglutamine and 2-Oxo-4-Methyl-3-Pentene-1,5-Dioic Acid in Leaves and Stem of Tulip Plants. Soil Sci Plant Nutr 1988;34:613-20.
Ohyama T, Komiyama S, Ohtake N, Sueyoshi K, Teixeira da Silva JA, Ruamrungsri S. Physiology and Genetics of Carbon and Nitrogen Metabolism in Tulip. Floriculture, Ornamental and Plant Biotechnology: Global Science Books, UK; 2006.
V. Rieke, K. Vigen, G. Sommer, B. Daniel, J. Pauly, K. Butts, Referenceless PRF Shift thermometry, MRM, 2004 Jun;51(6):1223-31.
Schroeder MA, Swietach P, Atherton HJ, et al. Measuring intracellular pH in the heart using hyperpolarized carbon dioxide and bicarbonate: a 13C and 31P magnetic resonance spectroscopy study. Cardiovascular research 2010;86:82-91.
Stodola FH, Shotwell OL, Lockwood LB. Zymonic Acid, a New Metabolic Product of Some Yeasts Grown in Aerated Culture .1. Structure Studies. J Am Chem Soc 1952;74:5415-8.
Vavere AL, Biddlecombe GB, Spees WM, et al. A Novel Technology for the Imaging of Acidic Prostate Tumors by Positron Emission Tomography. Cancer Res 2009;69:4510-6.
Zhang X, Lin Y, Gillies RJ. Tumor pH and its measurement. Journal of nuclear medicine : official publication, Society of Nuclear Medicine 2010;51:1167-70.

## Claims

1. Use of a compound with at least one pH-sensitive ¹³C chemical shift in magnetic resonance for determining pH and/or measuring pH changes by ¹³C magnetic resonance, wherein the compound is selected from
zymonic acid,
(*Z*)-4-methyl-2-oxopent-3-enedioic acid (OMPD),
analogs of zymonic acid,
analogs of OMPD,
and their hydrates, salts, solutions, stereoisomers,
wherein an analog of zymonic acid or OMPD is selected from
an ester, an ether, an amide, a fluorinated analog, deuterated analog or
wherein the zymonic acid analog is selected from 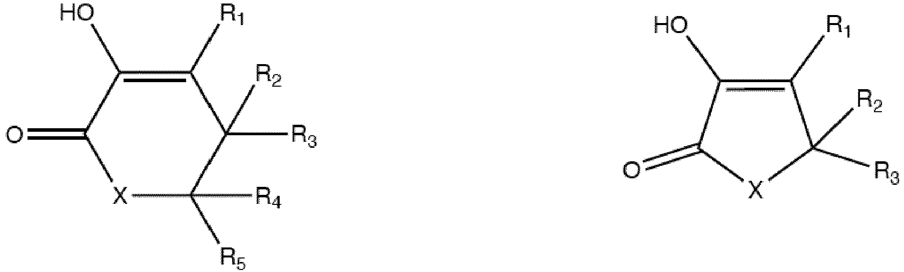 wherein X is selected from CR₆R₇, O, NR₆, S, and wherein R₁ to R₇ is, at each occurrence, independently selected from H, alkyl, halogen, CN, methoxy, carboxy, aryl, e.g. benzyl, wherein, preferably, one of R₂ and R₃ is carboxy.

2. The use of claim 1, wherein an ester analog of zymonic acid or OMPD is a methyl ester or wherein a fluorinated analog of zymonic acid or OMPD is a trifluoromethyl analog.

3. The use of claim 1 or 2, wherein the compound is ¹³C-labeled and exhibits at least one pH-sensitive ¹³C chemical shift,
and/or wherein the at least one pH-sensitive ¹³C chemical shift is pH-sensitive in a physiological and/or pathological pH range, preferably from pH 5 to pH 9.

4. The use of any one of claim s 1 to 3, wherein the compound furthermore exhibits at least one chemical shift that is not pH-sensitive, preferably at least one pH-insensitive ¹³C chemical shift.

5. The use of any one of claims 1 to 4, wherein the compound is hyperpolarized, preferably by dynamic nuclear polarization (DNP),
and/or wherein the compound has a pKₐ value in a physiological and/or pH range, preferably from 5 to 9, and/or the carbon(s) belonging to the pH-sensitive ¹³C chemical shift(s) of the compound exhibit(s) a long longitudinal relaxation time T₁.

6. The use of any one of claims 1 to 5, comprising the further use of a reference compound, wherein, preferably, the reference compound is a compound which does not exhibit pH-sensitive ¹³C chemical shift(s) in an NMR spectrum, preferably the reference compound is ¹³C-labeled and preferably exhibits at least one pH-insensitive ¹³C chemical shift.

7. A method of *in vivo* imaging by magnetic resonance imaging (MRI), magnetic resonance spectroscopy (MRS) or magnetic resonance tomography (MRT) using the compound as defined by any of claims 1 to 6.

8. The compound of any of claims 1 to 6 for use in an *in vivo* method for diagnosing and/or monitoring treatment of a disease causing changes in pH by ¹³C magnetic resonance, wherein a disease causing changes in pH is preferably selected from cancers, inflammation, ischemia, renal failure and chronic obstructive pulmonary disease

9. A compound according to claim 8 for use according to claim 8, wherein the imaging is
real-time,
preferably comprising the resolution of the spatial pH distribution,
more preferably comprising the use of frequency encoding techniques, such as chemical shift imaging (CSI) and phase sensitive encodings of chemical shifts.

10. Use of the compound of any of claims 1 to 6 as pH sensor for *in vitro* diagnosing and/or monitoring treatment of a disease causing changes in pH by ¹³C magnetic resonance, wherein a disease causing changes in pH is preferably selected from cancers, inflammation, ischemia, renal failure and chronic obstructive pulmonary disease

11. Use according to claim 10, wherein the imaging is real-time,
preferably comprising the resolution of the spatial pH distribution,
more preferably comprising the use of frequency encoding techniques, such as chemical shift imaging (CSI) and phase sensitive encodings of chemical shifts.

12. Use of the compound of any of claims 1 to 6 as pH sensor for *in vitro* NMR-spectroscopy,
preferably comprising response-to-treatment monitoring of treatments applied to cell lines.

13. An *in vitro* method for determining pH and/or measuring pH changes,
comprising the steps of
(i) providing a sample,
(ii) adding a compound with at least one pH-sensitive chemical shift of any of claims 1 to 6 to the sample,
(iii) performing magnetic resonance imaging (MRI) or magnetic resonance spectroscopy (MRS) and thereby determining the pH or pH changes of or in the sample by obtaining a chemical shift difference between at least one pH-sensitive chemical shift of the compound and a pH-independent chemical shift, such pH-independent chemical shift acting as a reference chemical shift, or by measurement of the absolute chemical shift, or by measuring chemical shift differences involving at least one pH-sensitive shift.
wherein the sample is preferably a cell culture sample (such as derived from a human or non-human body, *ex vivo* tissue, cell culture),
and/or wherein step (iii) is preferably carried out in an MRI scanner machine with MRS or MRSI capabilities or in a NMR spectrometer.

## Patentansprüche

1. Verwendung einer Verbindung mit mindestens einer pH-sensitiven ¹³C chemischen Verschiebung in der Magnetresonanz zur Bestimmung des pH und/oder Messung von pH-Änderungen mittels ¹³C Magnetresonanz,
wobei die Verbindung ausgewählt ist aus
Zymonsäure,
(*Z*)-4-Methyl-2-oxopent-3-andensäure (OMPD),
Analoga der Zymonsäure,
Analoga von OMPD,
und deren Hydrate, Salze, Lösungen, Stereoisomere,
wobei ein Analogon der Zymonsäure oder OMPD ausgewählt ist aus
einem Ester, einem Ether, einem Amid, einem fluorinierten Analogon, deuterierten Analogon oder
wobei das Zymonsäure-Analogon ausgewählt ist aus 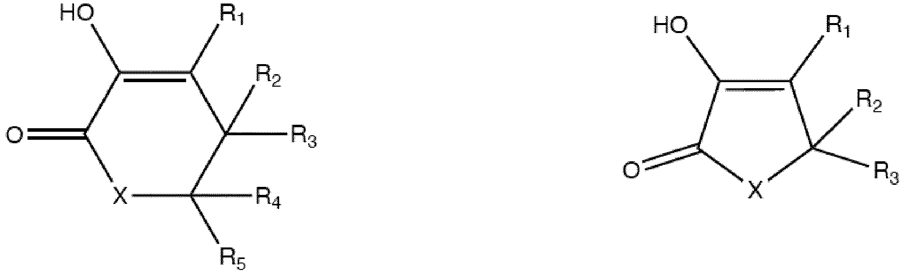 wobei X ausgewählt ist aus CR₆R₇, O, NR₆, S, und wobei R₁ bis R₇ bei jeder Gelegenheit unabhängig ausgewählt ist aus H, Alkyl, Halogen, CN, Methoxy, Carboxy, Aryl, beispielsweise Benzyl, wobei einer von R₂ und R₃ bevorzugt Carboxy ist.

2. Die Verwendung gemäß Anspruch 1, wobei ein Ester-Analogon der Zymonsäure oder von OMPD ein Methylester ist, oder wobei ein fluoriniertes Analogon der Zymonsäure oder von OMPD ein Trifluormethyl-Analogon ist.

3. Die Verwendung gemäß Anspruch 1 oder 2, wobei die Verbindung ¹³C-markiert ist und mindestens eine pH-sensitive ¹³C chemische Verschiebung aufweist,
und/oder wobei die mindestens eine pH-sensitive ¹³C chemische Verschiebung pH-sensitiv in einem physiologischen und/oder pathologischen pH Bereich ist, bevorzugt von pH 5 bis pH 9.

4. Die Verwendung gemäß einem der Ansprüche 1 bis 3, wobei die Verbindung weiterhin mindestens eine chemische Verschiebung aufweist, die nicht pH-sensitiv ist, bevorzugt mindestens eine pH-insensitive ¹³C chemische Verschiebung.

5. Die Verwendung gemäß einem der Ansprüche 1 bis 4, wobei die Verbindung hyperpolarisiert ist, bevorzugt durch dynamische Kernpolarisierung ("dynamic nuclear polarization") (DNP),
und/oder wobei die Verbindung einen pKₐ Wert in einem physiologischen und/oder pH Bereich hat, bevorzugt von 5 bis 9, und/oder der Kohlenstoff oder die Kohlenstoffe, der/die zur/zu den pH-sensitiven ¹³C chemische/n Verschiebung/en der Verbindung gehören, weist/weisen eine lange longitudinale Relaxationszeit T₁ auf.

6. Die Verwendung gemäß einem der Ansprüche 1 bis 5, umfassend die weitere Verwendung einer Referenz-Verbindung,
wobei die Referenz-Verbindung bevorzugt eine Verbindung ist, welche keine pH-sensitive ¹³C chemische Verschiebung(en) in einem NMR-Spektrum aufweist, bevorzugt ist die Referenz-Verbindung ¹³C-markiert und weist bevorzugt mindestens eine pH-insensitive ¹³C chemische Verschiebung auf.

7. Ein Verfahren zur *in vivo* Bildgebung durch Magnetresonanz-Imaging (MRI), Magnetresonanz-Spektroskopie (MRS) oder Magnetresonanz-Tomographie (MRT) unter der Verwendung einer Verbindung, wie definiert in einem der Ansprüche 1 bis 6.

8. Die Verbindung gemäß einem der Ansprüche 1 bis 6 zur Verwendung in einem *in vivo* Verfahren zur Diagnose und/oder Monitoring der Behandlung einer Erkrankung, die Änderungen im pH verursacht, durch ¹³C Magnetresonanz,
wobei eine Erkrankung, die Änderungen im pH verursacht, bevorzugt ausgewählt ist aus Krebsarten, Inflammation, Ischämie, Nierenversagen und chronisch obstruktiver Lungenerkrankung.

9. Eine Verbindung gemäß Anspruch 8 zur Verwendung gemäß Anspruch 8, wobei die Bildgebung in Echtzeit erfolgt,
bevorzugt umfassend die Auflösung der räumlichen pH-Verteilung,
weiter bevorzugt umfassend die Verwendung von Frequenz-Kodierungs-Techniken, wie beispielsweise Chemcial Shift Imaging (CSI) und Phasen-sensitive Kodierungen von chemischen Verschiebungen.

10. Verwendung der Verbindung gemäß einem der Ansprüche 1 bis 6 als pH-Sensor für *in vitro* Diagnose und/oder Monitoring der Behandlung einer Erkrankung, die Änderungen im pH verursacht, durch ¹³C Magnetresonanz,
wobei eine Erkrankung, die Änderungen im pH verursacht, bevorzugt ausgewählt ist aus Krebsarten, Inflammation, Ischämie, Nierenversagen und chronisch obstruktiver Lungenerkrankung.

11. Die Verwendung gemäß Anspruch 10, wobei die Bildgebung in Echtzeit erfolgt, bevorzugt umfassend die Auflösung der räumlichen pH-Verteilung,
weiter bevorzugt umfassend die Verwendung von Frequenz-Kodierungs-Techniken, wie beispielsweise Chemcial Shift Imaging (CSI) und Phasen-sensitive Kodierungen von chemischen Verschiebungen.

12. Verwendung der Verbindung gemäß einem der Ansprüche 1 bis 6 als pH-Sensor für *in vitro* NMR-Spektroskopie,
bevorzugt umfassend Reaktion auf Behandlung ("response-to-treatment") Monitoring von Behandlungen, die auf Zell-Linien angewandt werden.

13. Ein *in vitro* Verfahren zur Bestimmung des pH und/oder Messung von pH-Änderungen, umfassend die Schritte
(i) zur Verfügung stellen einer Probe,
(ii) Addieren einer Verbindung mit mindestens einer pH-sensitiven ¹³C chemischen Verschiebung gemäß einer der Ansprüche 1 bis 6 zu der Probe,
(iii) Durchführen von Magnetresonanz-Imaging (MRI) oder Magnetresonanz-Spektroskopie (MRS) und dabei Bestimmen des pH oder von pH-Änderungen von der Probe oder in der Probe durch Erhalten einer Differenz der chemischen Verschiebung zwischen mindestens einer pH-sensitiven chemischen Verschiebung der Verbindung und einer pH-unabhängigen chemischen Verschiebung, wie beispielsweise pH-unabhängige chemische Verschiebung fungierend als Referenz der chemischen Verschiebung, oder durch Messen der absoluten chemischen Verschiebung, oder durch Messen von Differenzen der chemischen Verschiebung, mindestens eine pH-sensitive Verschiebung einschließend,
wobei die Probe bevorzugt eine Zellkultur-Probe ist (wie abgeleitet von einem menschlichen oder nicht-menschlichen Körper, *ex vivo* Gewebe, Zellkultur),
und/oder wobei Schritt (iii) bevorzugt in einer MRI Scanner-Maschine mit MRS oder MRSI Fähigkeiten oder in einem NMR Spektrometer durchgeführt wird.

## Revendications

1. Utilisation d'un composé ayant au moins un déplacement chimique en ¹³C sensible au pH en résonance magnétique pour déterminer un pH et/ou mesurer des changements de pH par résonance magnétique ¹³C,
dans laquelle le composé est sélectionné parmi
l'acide zymonique,
l'acide (*Z*)-4-méthyl-2-oxopent-3-ènedioïque (OMPD),
les analogues de l'acide zymonique,
les analogues de OMPD,
et leurs hydrates, sels, solutions, stéréo-isomères,
dans laquelle un analogue de l'acide zymonique ou de OMPD est sélectionné parmi
un ester, un éther, un amide, un analogue fluoré, un analogue deutéré ou
dans lequel l'analogue de l'acide zymonique est sélectionné parmi 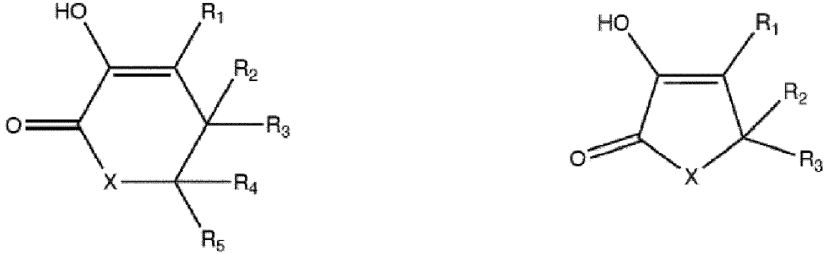
dans laquelle X est sélectionné parmi CR₆R₇, O, NR₆, S, et dans laquelle R₁ à R₇ est, à chaque occurrence, sélectionné indépendamment parmi H, un alkyle, un halogène, CN, un méthoxy, un carboxy, un aryle, par exemple un benzyle, dans laquelle, de préférence, un parmi R₂ et R₃ est un carboxy.

2. Utilisation selon la revendication 1, dans laquelle un analogue ester de l'acide zymonique ou de OMPD est un ester de méthyle ou dans laquelle un analogue fluoré de l'acide zymonique ou de OMPD est un analogue trifluorométhylé.

3. Utilisation selon la revendication 1 ou 2, dans laquelle le composé est marqué par ¹³C et présente au moins un déplacement chimique en ¹³C sensible au pH,
et/ou dans laquelle l'au moins un déplacement chimique en ¹³C sensible au pH est sensible au pH dans une plage de pH physiologique et/ou pathologique, de préférence de pH 5 à pH 9.

4. Utilisation selon l'une quelconque des revendications 1 à 3, dans laquelle le composé présente en outre au moins un déplacement chimique qui n'est pas sensible au pH, de préférence au moins un déplacement chimique en ¹³C insensible au pH.

5. Utilisation selon l'une quelconque des revendications 1 à 4, dans laquelle le composé est hyperpolarisé, de préférence par polarisation nucléaire dynamique (DNP),
et/ou dans laquelle le composé présente une valeur de pKₐ dans une plage physiologique et/ou de pH, de préférence de 5 à 9, et/ou le(s) carbone(s) appartenant au (x) déplacement(s) chimique(s) en ¹³C sensible(s) au pH du composé présente(nt) un temps de relaxation longitudinale T₁ long.

6. Utilisation selon l'une quelconque des revendications 1 à 5, comprenant l'utilisation supplémentaire d'un composé de référence, dans laquelle, de préférence, le composé de référence est un composé qui ne présente pas de déplacement chimique en ¹³C sensible au pH sur un spectre RMN, de préférence le composé de référence est marqué par ¹³C et de préférence présente au moins un déplacement chimique en ¹³C insensible au pH.

7. Procédé d'imagerie *in vivo* par imagerie par résonance magnétique (IRM), spectroscopie par résonance magnétique (SRM) ou tomographie par résonance magnétique (TRM) utilisant le composé tel que défini dans l'une quelconque des revendications 1 à 6.

8. Composé selon l'une quelconque des revendications 1 à 6 pour une utilisation dans un procédé *in vivo* pour diagnostiquer et/ou surveiller un traitement d'une maladie provoquant des changements de pH par résonance magnétique ¹³C,
dans lequel une maladie provoquant des changements de pH est sélectionnée de préférence parmi les cancers, une inflammation, une ischémie, une insuffisance rénale et une bronchopneumopathie chronique obstructive.

9. Composé selon la revendication 8 pour une utilisation selon la revendication 8, dans lequel l'imagerie est en temps réel,
de préférence comprenant la résolution de la distribution spatiale du pH,
de manière davantage préférée comprenant l'utilisation de techniques de codage en fréquence, telles que l'imagerie par déplacement chimique (CSI) et les codages sensibles à la phase de déplacements chimiques.

10. Utilisation du composé selon l'une quelconque des revendications 1 à 6 comme capteur de pH pour diagnostiquer et/ou surveiller *in vitro* un traitement d'une maladie provoquant des changements de pH par résonance magnétique ¹³C,
dans laquelle une maladie provoquant des changements de pH est sélectionnée de préférence parmi les cancers, une inflammation, une ischémie, une insuffisance rénale et une bronchopneumopathie chronique obstructive.

11. Utilisation selon la revendication 10, dans laquelle l'imagerie est en temps réel,
de préférence comprenant la résolution de la distribution spatiale du pH,
de manière davantage préférée comprenant l'utilisation de techniques de codage en fréquence, telles que l'imagerie par déplacement chimique (CSI) et les codages sensibles à la phase de déplacements chimiques.

12. Utilisation du composé selon l'une quelconque des revendications 1 à 6 comme capteur de pH pour spectroscopie RMN *in vitro,*
de préférence comprenant une surveillance de réponse au traitement de traitements appliqués à des lignées cellulaires.

13. Procédé *in vitro* pour déterminer un pH et/ou mesurer des changements de pH,
comprenant les étapes de
(i) fourniture d'un échantillon,
(ii) ajout d'un composé ayant au moins un déplacement chimique sensible au pH selon l'une quelconque des revendications 1 à 6 à l'échantillon,
(iii) mise en oeuvre d'une imagerie par résonance magnétique (IRM) ou d'une spectroscopie par résonance magnétique (SRM) et ainsi la détermination du pH ou des changements de pH de ou dans l'échantillon par l'obtention d'une différence de déplacements chimiques entre au moins un déplacement chimique sensible au pH du composé et un déplacement chimique indépendant du pH, un tel déplacement chimique indépendant du pH jouant le rôle de déplacement chimique de référence, ou par la mesure du déplacement chimique absolu, ou par la mesure de différences de déplacements chimiques impliquant au moins un déplacement sensible au pH,
dans lequel l'échantillon est de préférence un échantillon de culture cellulaire (tel que dérivé d'un corps humain ou non humain, d'un tissu *ex vivo,* d'une culture cellulaire),
et/ou dans lequel l'étape (iii) est de préférence réalisée dans une machine de balayage IRM ayant des capacités SRM ou ISRM ou dans un spectromètre RMN.
